# EUROPÄISCHE PATENTSCHRIFT

(11) **EP 1 315 697 B1**
(45) Veröffentlichungstag und Bekanntmachung des Hinweises auf die Patenterteilung: **22.03.2006**
(21) Anmeldenummer: 01960682.1
(22) Anmeldetag: 21.08.2001
(51) Int. Cl.: C07C 317/24, C07C 49/84, A01N 41/10, A01N 35/06

(54) **HERBIZID WIRKSAME BENZOYLCYCLOHEXANDIONE**
HERBICIDAL BENZOYL CYCLOHEXANE DIONE
BENZOYLECYCLOHEXANEDIONE A ACTION HERBICIDE

(30) Priorität: 01.09.2000 DE 10043074
(43) Veröffentlichungstag der Anmeldung: 04.06.2003
(73) Patentinhaber: Bayer CropScience GmbH, 65929 Frankfurt (DE)
(72) Erfinder: SEITZ,Thomas, 68514 Viernheim (DE); VAN ALMSICK, Andreas, 61184 Karben (DE); WILLMS, Lothar, 65719 Hofheim (DE); BIERINGER, Hermann, 65817 Eppstein (DE); AULER, Thomas, 65812 Bad Soden (DE); MENNE, Hubert, 65719 Hofheim Ts. (DE)
(86) Internationale Anmeldenummer: PCT/EP2001/009600
(87) Internationale Veröffentlichungsnummer: WO 2002/018331

(56) Entgegenhaltungen:
- EP-A- 0 579 223
- WO-A-98/41089
- GB-A- 2 061 913
- US-A- 4 440 940
- US-A- 4 986 845
- US-A- 5 712 298
- US-A- 5 935 978
- PATENT ABSTRACTS OF JAPAN vol. 1998, no. 08, 30. Juni 1998 (1998-06-30) -& JP 10 059929 A (HOKKO CHEMICAL INDUSTRIES), 3. März 1998 (1998-03-03)
- PATENT ABSTRACTS OF JAPAN vol. 018, no. 680 (C-1291), 21. Dezember 1994 (1994-12-21) & JP 06 271562 A (HOKKO CHEMICAL INDUSTRIES), 27. September 1994 (1994-09-27)

## Beschreibung

Die Erfindung betrifft das technische Gebiet der Herbizide, insbesondere das der Herbizide zur selektiven Bekämpfung von Unkräutern und Ungräsem in Nutzpflanzenkulturen.

Aus verschiedenen Schriften ist bereits bekannt, daß bestimmte Benzoylcyclohexandione, auch solche, die in 3-Position des Phenylrings beispielsweise durch einen cyclischen Rest substituiert sind, herbizide Eigenschaften besitzen. WO 99/10327 offenbart Benzoylcyclohexandione, die in 3-Position des Phenylrings einen über eine Kette von Kohlenstoffatomen gebundenen heterocyclischen Rest, enthaltend mindestens ein Stickstoffatom, tragen. In WO 00/21924 werden Benzoylcyclohexandione genannt, die in 3-Position des Phenylrings einen über eine Kette von Kohlenstoffatomen gebundenen hetero- oder carbocyclischen Rest tragen.

Die Anwendung der aus diesen Schriften bekannten Verbindungen ist jedoch häufig in der Praxis mit Nachteilen verbunden. So ist die herbizide Wirksamkeit der bekannten Verbindungen nicht immer ausreichend, oder bei ausreichender herbizider Wirksamkeit werden unerwünschte Schädigungen der Nutzpflanzen beobachtet. Aufgabe der vorliegenden Erfindung war daher die Bereitstellung von herbizid wirksamen Verbindungen mit - gegenüber den aus dem Stand der Technik bekannten Verbindungen- verbesserten herbiziden Eigenschaften.

Es wurde nun gefunden, daß Benzoylcyclohexandione, die in 3-Position des Phenylrings bestimmte über ein Sauerstoff-, Stickstoff- oder Schwefelatom gebundene cyclische Reste tragen, als Herbizide besonders gut geeignet sind. Ein Gegenstand der vorliegenden Erfindung sind daher Verbindungen der Formel (I) oder deren Salze 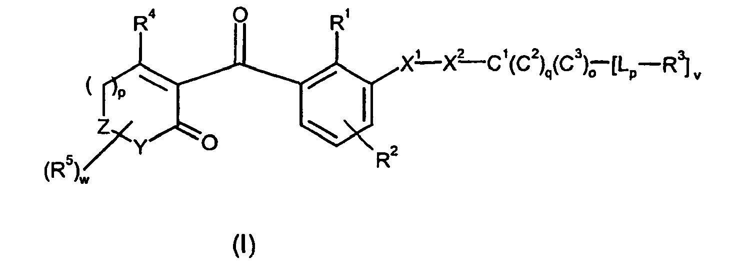 worin
- X¹: eine divalente Einheit aus der Gruppe O, S(O)ₙ, NH, N[Lp - R³];
- X²: eine geradkettige oder verzweigte, durch w Halogenatome und durch k Reste [Lₚ-R³] substituierte (C₁-C₆)-Alkylen-, (C₂-C₆)-Alkenylen- oder (C₂-C₆)-Alkinylenkette;
C¹(C²)_{q}(C³)ₒ einen mono-, bi- oder tricyclischer Rest, wobei
a) die Ringe C¹, C² und C³ jeweils für einen 3- bis 8-gliedrigen, gesättigten oder teilgesättigten Ring aus der Gruppe Cycloalkyl, Cycloalkenyl, Oxiranyl und Oxetanyl stehen,
b) die Ringe C¹, C² und C³ jeweils untereinander über ein oder zwei gemeinsame Atome verknüpft sind;

- R¹ und R²: unabhängig voneinander Wasserstoff, Mercapto, Nitro, Cyano, Halogen, Thiocyanato, (C₁-C₆)-Alkyl-CO-O, (C₁-C₆)-Alkyl-S(O)ₙ-O, (C₁-C₆)-AlkylS(O)ₙ, Di-(C₁-C₆)-Alkyl-NH-SO₂, (C₁-C₆)-Alkyl-SO₂-NH, (C₁-C₆)-Alkyl-NH-CO, (C₁₋C₆)-Alkyl-SO₂-[(C₁-C₆)-Alkyl]amino, (C₁-C₆)-Alkyl-CO-((C₁-C₆)-Alkyl)-amino, 1,2,4,-Triazol-1-yl, (C₁-C₆)-Alkyl-O-CH₂₉ (C₁-C₆)-Alkyl-S(O)ₙ-CH₂, (C₁-C₆)-Alkyl-NH-CH₂, [(C₁-C₆)-Alkyl]₂N-CH₂, 1,2,4,-Triazol-1-yl-CH₂, durch v Reste aus der Gruppe Cyano, Nitro und Halogen substituiertes (C₁-C₆)-Alkyl-(D)ₚ, (C₂-C₆)-Alkenyl-(D)p, (C₂₋C₆)-Alkinyl-(D)p, (C₃-C₉)-Cycloalkyl-(D)ₚ, (C₃-C₉)-Cycloalkenyl-(D)ₚ, (C₁-C₆)-Alkyl-(C₃₋C₉)-Cycloalkyl-(D)ₚ oder (C₁-C₆)-Alkyl-(C₃C₉)-Cycloalkenyl-(D)ₚ;
- R³: Wasserstoff, Hydroxy, Halogen, Mercapto, Amino, Nitro, ein kohlenstoffhaltiger Rest oder, falls p in X¹ für null steht, Oxo, NR⁸, N-OR⁸ oder N-NR⁸R9;
- D: Sauerstoff oder Schwefel;
- L: jeweils geradkettiges oder verzweigtes Aₚ-[C(R⁶)₂]_{w}-[Aₚ-C(R⁶)₂]ₓ-Aₚ oder Ap-M-Ap;
mit der Maßgabe, daß 2 oder 3 der Laufzahlen p, w und x nicht gleichzeitig null sein sollen;
- A: eine divalente Einheit aus der Gruppe O, S(O)ₙ, NH, N-(C₁-C₆)-Alkyl, N-(C₂-C₆)-Alkenyl und N-(C₂-C₆)-Alkinyl;
- M: durch w Reste R⁶ substituiertes (C₁-C₆)-Alkylen, (C₂-C₆)-Alkenylen oder (C₂-C₆)-Alkinylen;
- R⁴: OR⁷, (C₁-C₄)-Alkylthio, Halogen-(C₁-C₄)-alkylthio, (C₁-C₄)-Alkenylthio, Halogen-(C₂-C₄)-alkenylthio, (C₂-C₄)-Alkinylthio, Halogen-(C₂-C₄)-alkinylthio, (C₂-C₄)-Alkylsulfinyl, Halogen-(C₂-C₄)-alkylsulfinyl, (C₂-C₄)-Alkenylsulfinyl, Halogen-(C₂-C₄)-alkenylsulfinyl, (C₂-C₄)-Alkinylsulfinyl, Halogen-(C₂-C₄)-alkinylsulfinyl, (C₁-C₄)-Alkylsulfonyl, Halogen-(C₁-C₄)-alkylsulfonyl, (C₂-C₄)-Alkenylsulfonyl, Halogen-(C₂-C₄)-alkenylsulfonyl, (C₂-C₄)-Alkinylsulfonyl, Halogen-(C₂-C₄)-alkinylsulfonyl, Cyano, Cyanato, Thiocyanato, Halogen oder Phenylthio;
- R⁵: Wasserstoff, Tetrahydropyranyl-3, Tetrahydropyranyl-4, Tetrahydrothiopyranyl-3, (C₁-C₄)-Alkyl, (C₃-C₈)-Cycloalkyl, (C₁-C₄)-Alkoxy, (C₁-C₄)-Alkoxy-(C₁-C₄)-alkyl, (C₁-C₄)-Alkylcarbonyl, (C₁-C₄)-Alkoxycarbonyl, (C₁-C₄)-Alkylthio, Phenyl, wobei die acht letztgenannten Gruppen durch v Reste aus der Gruppe Halogen, (C₁-C₄)-Alkylthio und (C₁-C₄)-Alkoxy substituiert sind, oder zwei an einem gemeinsamen Kohlenstoffatom gebundene Reste R⁵ bilden eine Kette aus der Gruppe OCH₂CH₂O, OCH₂CH₂CH₂O, SCH₂CH₂S und SCH₂CH₂CH₂S, wobei diese durch w Methylgruppen substituiert ist, oder zwei an direkt benachbarten Kohlenstoffatomen gebundene Reste R⁵ bilden mit den sie tragenden Kohlenstoffatomen einen durch w Reste aus der Gruppe Halogen, (C₁-C₄)-Alkyl, (C₁-C₄)-Alkylthio und (C₁-C₄)-Alkoxy substituierten 3- bis 6-gliedrigen Ring;
- R⁶: (C₁-C₄)-Alkyl, Halogen, Cyano oder Nitro;
- R⁷: Wasserstoff, (C₁-C₄)-Alkyl, Halogen-(C₁-C₄)-alkyl, (C₁-C₄)-Alkoxy-(C₁-C₄)-alkyl, Formyl, (C₁-C₄)-Alkylcarbonyl, (C₁-C₄)-Alkoxycarbonyl, (C₁-C₄)-Alkylaminocarbonyl, Di-(C₁-C₄)-alkylaminocarbonyl, (C₁-C₄)-Alkylsulfonyl, Halogen-(C₁-C₄)-alkylsulfonyl, Benzoyl oder Phenylsulfonyl, wobei die beiden letztgenannten Gruppen durch v Reste aus der Gruppe (C₁-C₄)-Alkyl, Halogen-(C₁-C₄)-alkyl, (C₁-C₄)-Alkoxy, Halogen-(C₁-C₄)-alkoxy, Halogen, Cyano und Nitro substituiert sind;
- R⁸: Wasserstoff, (C₁-C₄)-Alkyl. (C₁-C₄)-Alkoxy, (C₂-C₄)-Alkenyl, (C₂-C₄)-Alkinyl, (C₃-C₈)-Cycloalkyl, Aryl, Aryl-(C₁-C₆)-alkyl, Heteroaryl, Heterocyclyl, Halogen-(C₁-C₄)-alkyl;
- R⁹: Wasserstoff, (C₁-C₄)-Alkyl, (C₂-C₄)-Alkenyl, (C₂-C₄)-Alkinyl, (C₃-C₉)-Cycloalkyl, Aryl, Aryl-(C₁-C₆)-alkyl, Heteroaryl, Heterocyclyl, Halogen-(C₁-C₄)-alkyl, oder sofern R⁸ und R⁹ an einem Atom oder an zwei direkt benachbarten Atomen gebunden sind, bilden sie gemeinsam mit den sie bindenden Atomen einen gesättigten, teilweise oder vollständig ungesättigten fünf bis sechsgliedrigen Ring, der p Heteroatome aus der Gruppe Sauerstoff, Stickstoff und Schwefel enthält;
- Y: eine divalente Einheit aus der Gruppe O, S, N-H, N-(C₁-C₄)-Alkyl, CHR⁵ und C(R⁵)₂;
- Z: eine divalente Einheit aus der Gruppe O, S, SO, SO₂, N-H, N-(C₁-C₄)-Alkyl, CHR⁵ und C(R⁵)₂;
- m und n: jeweils 0, 1 oder 2;
- o, p und q: jeweils 0 oder 1;
- w und x: jeweils 0,1, 2, 3 oder 4;
- v: 0, 1, 2 oder 3 bedeuten.

Zahlreiche erfindungsgemäße Verbindungen der Formel (I) können in Abhängigkeit von äußeren Bedingungen, wie Lösungsmittel und pH-Wert, in unterschiedlichen tautomeren Strukturen auftreten. Je nach Art der Substituenten enthalten die Verbindungen der allgemeinen Formel (I) ein acides Proton, das durch Umsetzung mit einer Base entfernt werden kann. Als Basen eignen sich beispielsweise Hydride, Hydroxide und Carbonate von Lithium, Natrium, Kalium, Magnesium und Calcium sowie Ammoniak und organische Amine wie Triethylamin und Pyridin. Solche Salze sind ebenfalls Gegenstand der Erfindung.

In allen nachfolgend genannten Formeln haben die Substituenten und Symbole, sofern nicht anders definiert, dieselbe Bedeutung wie unter Formel (I) beschrieben.

In Formel (I) und allen nachfolgenden Formeln können kettenförmige kohlenstoffhaltige Reste wie Alkyl, Alkoxy, Halogenalkyl, Halogenalkoxy, Alkylamino und Alkylthio sowie die entsprechenden ungesättigten und/oder substituierten Reste im Kohlenstoffgerüst wie Alkenyl und Alkinyl jeweils geradkettig oder verzweigt sein. Wenn nicht speziell angegeben, sind bei diesen Resten die niederen Kohlenstoffgerüste, z.B. mit 1 bis 6 C-Atomen bzw. bei ungesättigten Gruppen mit 2 bis 4 C-Atomen, bevorzugt. Alkylreste, auch in den zusammengesetzten Bedeutungen wie Alkoxy, Halogenalkyl usw., bedeuten z.B. Methyl, Ethyl, n- oder i-Propyl, n-, i-, t-oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste; Alkenyl bedeutet z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Propargyl, But-2-in-1-yl, But-3-in-1-yl, 1-Methyl-but-3-in-1-yl. Die Mehrfachbindung kann sich in beliebiger Position des ungesättigten Rests befinden.

Cycloalkyl bedeutet, sofern nicht speziell angegeben, ein carbocyclisches, gesättigtes Ringsystem mit drei bis acht C-Atomen, z.B. Cyclopropyl, Cyclopentyl oder Cyclohexyl. Analog bedeutet Cycloalkenyl eine monocyclische Alkenylgruppe mit drei bis acht Kohlenstoffringgliedern, z.B. Cyclopentyl, Cyclobutenyl und Cyclohexenyl, wobei sich die Doppelbindung an beliebiger Position befinden kann. Wenn o und/oder q gleich 1 ist, liegt der Rest C¹(C²)_{q}(C³)ₒ als bi- oder tricyclischer Rest vor. Beispiele dafür sind Adamantyl, Bicyclo[4.1.0]heptanyl, Bicyclo[3.2.0]heptanyl, Bicyclo[4.2.0]octanyl, Bicyclo[3.3.0]octanyl, Bicyclo[4.3.0]nonanyl, Bicyclo[4.3.0]non-1en-yl, Bicyclo[2.2.1]hept-2-en-yl, Bicyclo[2.2.2]oct-2-en-yl, Bicyclo[3.1.1]hept-2-en-yl, Bicyclo[3.3.1]non-2-en-yl, Spiro[2.2]pentanyl und Dispiro[2.2.1]heptanyl.

Im Falle einer zweifach substituierten Aminogruppe, wie Dialkylamino, können diese beiden Substituenten gleich oder verschieden sein.

Halogen bedeutet Fluor, Chlor, Brom oder lod. Halogenalkyl, -alkenyl und -alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. CF₃, CHF₂, CH₂F, CF₃CF₂, CH₂FCHCl, CCl₃, CHCl₂, CH₂CH₂Cl; Halogenalkoxy ist z.B. OCF₃, OCHF₂, OCH₂F, CF₃CF₂O, OCH₂CF₃ und OCH₂CH₂Cl; entsprechendes gilt für Halogenalkenyl und andere durch Halogen substituierte Reste.

Unter dem Begriff Heterocyclyl sind die Reste von drei- bis neungliedrigen, gesättigten, teilweise oder vollständig ungesättigen Heterocyclen zu verstehen, die ein bis drei Heteroatome ausgewählt aus der Gruppe Sauerstoff, Stickstoff und Schwefel enthalten. Die Verknüpfung kann, sofern chemisch möglich an beliebiger Position des Heterocyclus erfolgen. Bevorzugt steht Heterocyclyl für Aziridinyl, Oxiranyl, Tetrahydrofuranyl, Tetrahydropyranyl, Tetrahydrothienyl, Pyrrolidinyl, Isoxazolidinyl, Isoxazolinyl, Thiazolinyl, Thiazolidinyl, Pyrazolidinyl, Morpholinyl, Piperidinyl, Dioxolanyl, Dioxanyl, Piperazinyl, Oxepanyl, Azepanyl.

Heteroaryl steht für den Rest eines Heteroaromaten, der neben Kohlenstoff ringgliedem ein bis fünf Heteroatome aus der Gruppe Stickstoff, Sauerstoff und Schwefel enthält. Bevorzugt steht Heteroaryl für Furanyl, Thienyl, Pyrrolyl, Pyrazolyl, Imidazolyl, Oxazolyl, Thiazolyl, Isoxazolyl, isothiazolyl, 1,2,3-Triazolyl, 1,2,4-Triazolyl, 1,2,3-Oxadiazolyl, 1,2,4-Oxadiazotyl, 1,2,5-Oxadiazolyl, 1,3,4-Oxadiazolyl, 1,2,3-Thiadiazolyl, 1,2,4- Thiadiazolyl, 1,2,5-Thiadiazolyl, 1,3,4-Thiadiazolyl, Tetrazolyl, Pyridyl, Pyridazinyl, Pyrimidinyl, Pyrazinyl, 1,2,4-Triazinyl, 1,3,5-Triazinyl.

Aryl steht für einen aromatischen mono- oder polycyclischen Kohlenwasserstoffrest, z.B. Phenyl, Naphthyl, Biphenyl und Phenanthryl.

Ist eine Gruppe oder ein Rest mehrfach substituiert, so ist darunter zu verstehen, daß bei der Kombination der verschiedenen Substituenten die allgemeinen Grundsätze des Aufbaus chemischer Verbindungen zu beachten sind, d.h. daß nicht Verbindungen gebildet werden, von denen der Fachmann weiß, daß sie chemisch instabil oder nicht möglich sind. Dies gilt sinngemäß auch für die Verknüpfungen einzelner Reste.

Ist eine Gruppe oder ein Rest mehrfach durch andere Reste substituiert, so können diese anderen Reste gleich oder verschieden sein. Ist ein heterocyclischer Rest durch Hydroxy substituiert, so soll von dieser Definition auch die tautomere Form der Oxo-Gruppe umfasst sein.

Die Verbindungen der allgemeinen Formel (1) können je nach Art und Verknüpfung der Substituenten als Stereoisomere vorliegen. Sind beispielsweise eine oder mehrere Alkenylgruppen vorhanden, so können Diastereomere auftreten. Sind beispielsweise ein oder mehrere asymmetrische Kohlenstoffatome vorhanden, so können Enantiomere und Diastereomere auftreten. Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfaßt, jedoch nicht spezifisch definiert sind.

Für die Auswahl der Bedeutungen von "Y" und "Z" soll gelten, daß "Y" und "Z" nicht gleichzeitig jeweils für eine heteroatomige divalente Einheit stehen.

Unter einem kohlenstoffhaltigen Rest ist ein Rest mit mindestens einem Kohlenstoffatom und mehreren gleichen oder verschiedenen Atomen aus der Gruppe Wasserstoff, Halogen, Sauerstoff, Stickstoff, Schwefel und Phosphor zu verstehen. Insbesondere sind unter dieser Definition die folgenden Reste zu verstehen:
a) Cyano, Formyl;
b) durch w Reste aus der Gruppe Halogen, Cyano, Nitro Formyl, (C₁-C₄)-Alkyl, Halogen-(C₁-C₄)-alkyl, (C₁-C₄)-Alkoxy, Halogen-(C₁-C₄)-alkoxy, (C₁-C₄)-Alkylthio, Halogen-(C₁-C₄)-alkylthio und R¹⁰ substituiertes Aryl, ein bis vier Heteroatome aus der Gruppe Sauerstoff, Stickstoff und Schwefel enthaltendes mono- oder bicyclisches Heterocyclyl oder Heteroaryl;
c) durch w Reste aus der Gruppe Formyl, Halogen, Cyano, Nitro, (C₁-C₄)-Alkylamino, (C₁-C₄)-Dialkylamino, (C₁-C₄)-Alkoxycarbonyl, (C₁-C₄)-Alkylcarbonyl, (C₁-C₄)-Alkylcarbonyloxy, (C₁-C₄)-Alkyl, (C₂-C₄)-Alkenyl, (C₂-C₄)-Alkinyl, Halogen-(C₁-C₄)-alkyl, (C₂-C₄)-Halogenalkenyl, (C₂-C₄)-Halogenalkinyl, (C₁-C₄)-Alkylthio, Halogen-(C₁-C₄)-alkylthio, (C₁-C₄)-Alkoxy und Halogen-(C₁-C₄)-alkoxy substituiertes (R¹¹)(C₁-C₄)-Alkylamino, (R¹¹)₂-Amino, R¹¹-Oxycarbonyl, R¹¹-Carbonyl, R¹¹-Carbonyloxy; (C₁-C₆)-Alkyl, (C₂-C₆)-Alkenyl, (C₂-C₆)-Alkinyl, (C₁-C₆)-Alkoxy-(C₁-C₆)-alkyl, (C₂-C₆)-Alkenyloxy-(C₁-C₆)-alkyl, (C₂-C₆)-Alkinyloxy-(C₁-C₆)-alky), (C₃-C₉)-Cycloalkyl, (C₃-C₉)-Cyloalkenyl, (C-₂-C₆)-Alkenyl-(C₃-C₉)-cycloalkyl, (C₂-C₆)-Alkenyl-(C₃-C₉)-cycloalkyl-(C₁-C₆)-alkyl, (C₂-C₆)-Alkenyl-(C₃-C₉)-cycloalkenyl, (C₂-C₆)-Alkylenyl-(C₃-C₆)-cycloalkenyl-(C₁-C₆)-alkyl, (C₂-C₆)-Alkenyl-(C₃-C₉)-cycloalkyl, (C₂-C₆)-Alkenyl-(C₃-C₉)-cycloalkyl-(C₁-C₆)-alkyl, (C₂-C₆)-Alkenyl-(C₃-C₉)-cycloalkenyl, (C₂-C₆)-Alkenyl-(C₃-C₉)-cycloalkenyl-(C₁-C₆)-alkyl, (C₂-C₆)-Alkinyl-(C₃-C₉)-cycloalkyl, (C₂-C₆)-Alkinyl-(C₃-C₉)-cycloalkyl-(C₁-C₆)-alkyl, (C₂-C₆)-Alkinyl-(C₃-C₉)-cycloalkenyl, (C₂-C₆)-Alkinyl-(C₃-C₉)-cycloalkenyl-(C₁-C₆)-alkyl, (C₁-C₆)-Alkoxy oder (C₁-C₆)-Alkylthio;
d) ein Rest der Formel (Va) bis (Vz-3):

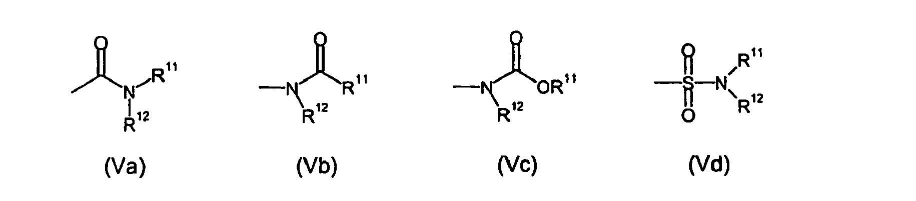

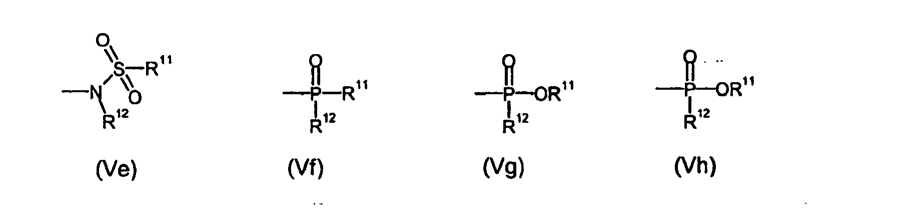

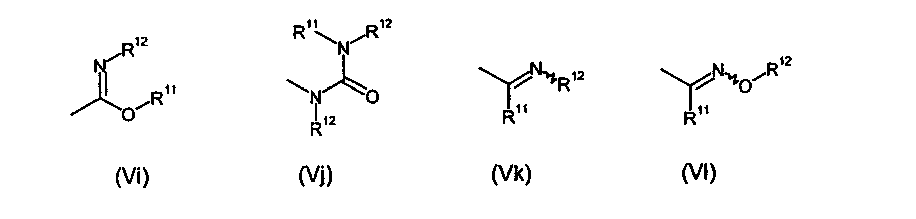

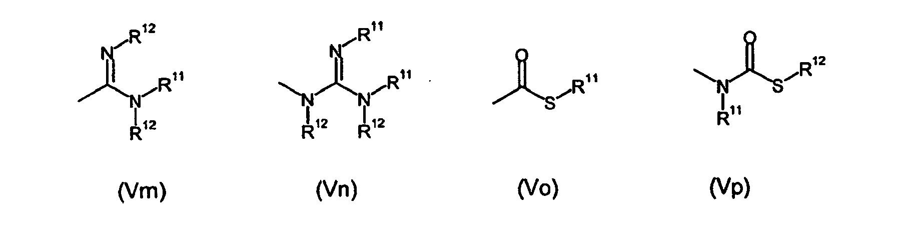

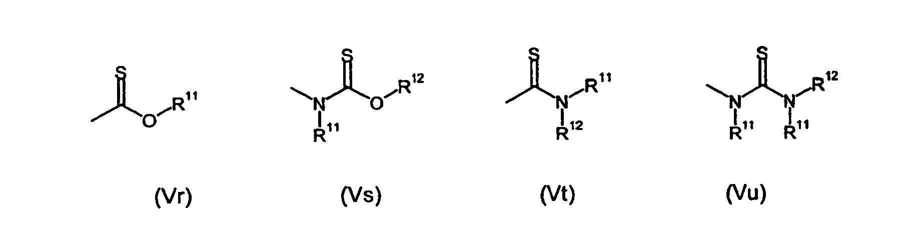

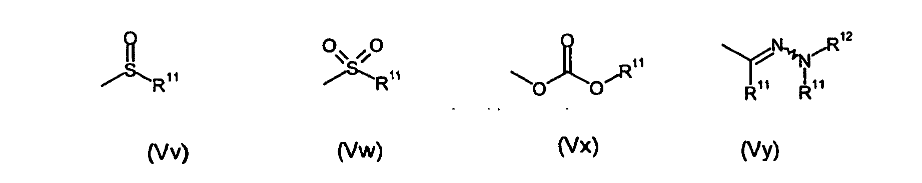

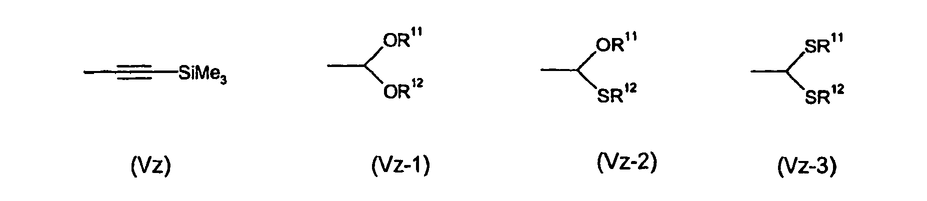

Darin bedeuten:
- R¹⁰: [(C₁-C₄)-Alkylen-O-(C₁-C₄)-alkylen]ₒ-O-(C₁-C₄)-alkyl, durch v Reste aus der Gruppe Halogen, Cyano und Nitro substituiertes (C₁-C₄)-alkyl, (C₂-C₄)-Alkenyl oder (C₂-C₄)-Alkinyl;
- R¹¹ und R¹²: unabhängig voneinander Wasserstoff, durch v Reste aus der Gruppe Halogen , Cyano und Nitro substituiertes (C1-C6)-Alkyl, (C₂-C₆)-Alkenyl, (C₂-C₆)-Alkinyl, (C₁-C₆)-Alkoxy-(C₁-C₆)-alkyl, (C₂-C₆)-Alkenyloxy-(C₁-C₆)-alkyl, (C₂-C₆)-Alkinyloxy-(C₁-C₆)-alkyl, (C₃-C₉)-Cycloalkyl, (C₃-C₉)-Cyloalkenyl, (C₁-C₆)-Alkyl-(C₃-C₉)-cycloalkyl, (C₁-C₆)-Alkyl-(C₃-C₉)-cycloalkyl-(C₁-C₆)-alkyl, (C₁-C₆)-Alkyl-(C₃-C₉)-cycloalkenyl, (C₁-C₆)-Alkyl-(C₃-C₉)-cycloalkenyl-(C₁-C₆)-alkyl, (C₂-C₆)-Alkenyl-(C₃-C₉)-cycloalkyl, (C₂-C₆)-Alkenyl-(C₃-C₉)-cycloalkyl-(C₁-C₆)-alkyl, (C₂-C₆)-Alkenyl-(C₃-C₉)-cycloalkenyl, (C₂-C₆)-Alkenyl-(C₃-C₉)-cycloalkenyl-(C₁-C₆)-alkyl, (C₂-C₆)-Alkinyl-cycloalkyl, (C₂-C₆)-Alkinyl-(C₃-C₉)-cycloalkyl-(C₁-C₆)-alkyl, (C₂-C₆)-Alkinyl-(C₃-C₉)-cycloalkenyl, (C₂-C₆)-Alkinyl-(C₃-C₉)-cycloalkenyl-(C₁-C₆)-alkyl, Aryl, Aryl-(C₁-C₆)-alkyl, Aryl-(C₂-C₆)-alkenyl, oder
R¹¹ und R¹² bilden gemeinsam mit den sie bindenden Atomen einen gesättigten, teilweise oder vollständig ungesättigten fünf- bis sechsgliedrigen Ring, der neben Kohlenstoffatomen p Heteroatome aus der Gruppe Sauerstoff, Stickstoff und Schwefel enthält;

Von näherem Interesse sind Verbindungen der allgemeinen Formel 1, worin
- X¹: eine divalente Einheit aus der Gruppe O, S und NH;
- R¹: Chlor, Brom, Fluor, Methyl, Ethyl, Cyano, Nitro, Halogen-(C₁-C₂)-alkyl;
- R²: Halogen, Halogen-(C₁-C₄)-alkyl, (C₁-C₄)-Alkylsulfenyl, (C₁-C₄)-Alkylsulfinyl, (C₁-C₄)-Alkylsulfonyl oder Nitro;
- R⁵: (C₁-C₄)-Alkyl, (C₃-C₈)-Cycloalkyl, (C₁-C₄)-Alkoxy, (C₁-C₄)-Alkoxy-(C₁-C₄)-alkyl, (C₁-C₄)-Alkylcarbonyl, (C₁-C₄)-Alkoxycarbonyl, (C₁-C₄)-Alkylthio, Phenyl, oder zwei an einem gemeinsamen Kohlenstoffatom gebundene Reste R⁵ bilden eine Kette aus der Gruppe OCH₂CH₂O, OCH₂CH₂CH₂O, SCH₂CH₂S und SCH₂CH₂CH₂S, wobei diese durch w Methylgruppen substituiert ist, oder zwei an direkt benachbarten Kohlenstoffatomen gebundene Reste R⁵ bilden eine Bindung oder bilden mit den sie tragenden Kohlenstoffatomen einen durch w Reste aus der Gruppe Halogen, (C₁-C₄)-Alkyl, (C₁-C₄)-Alkylthio und (C₁-C₄)-Alkoxy substituierten 3- bis 6-gliedrigen Ring;
- R⁸: Wasserstoff, (C₁-C₄)-Alkyl, (C₁-C₄)-Alkoxy, (C₂-C₄)-Alkenyl, (C₂-C₄)-Alkinyl, (C₃-C₈)-Cycloalkyl, Aryl, Aryl-(C₁-C₆)-alkyl, Halogen-(C₁-C₄)-alkyl;
- R⁹: Wasserstoff, (C₁-C₄)-Alkyl, oder sofern R⁸ und R⁹ an einem Atom oder an zwei direkt benachbarten Atomen gebunden sind, bilden sie gemeinsam mit den sie bindenden Atomen einen gesättigten, teilweise oder vollständig ungesättigten fünf-bis sechsgliedrigen Ring, der p Heteroatome aus der Gruppe Sauerstoff, Stickstoff und Schwefel enthält.

Von besonderem Interesse sind Verbindungen der allgemeinen Formel I, worin
- X²: eine geradkettige oder verzweigte, durch w Halogenatome substituierte (C₁-C₄)-Alkylen-, (C₂-C₄₋Alkenylen- oder (C₂-C₄)-Alkinylenkette;
- R³: a) Wasserstoff, Hydroxy, Halogen, Mercapto, Amino, Nitro, Cyano, Formyl,
b) durch w Reste aus der Gruppe Halogen, Cyano, (C₁-C₄)-Alkyl, Halogen-(C₁-C₄)-alkyl, (C₁-C₄)-Alkoxy, Halogen-(C₁-C₄)-alkoxy, (C₁-C₄)-Alkylthio, Halogen-(C₁-C₄)-alkylthio und R¹⁰ substituiertes Phenyl, Oxazolyl, Furanyl oder Tetrahydropyrrolyl,
c) durch v Reste aus der Gruppe Formyl, Halogen, Cyano, Nitro, (C₁-C₄)-Alkylamino, (C₁-C₄)-Dialkylamino, (C₁-C₄)-Alkoxycarbonyl, (C₁-C₄)-Alkylcarbonyl, (C₁-C₄)-Alkylcarbonyloxy, (C₁-C₄)-Alkyl, (C₂-C₄)-Alkenyl, (C₂-C₄)-Alkinyl, Halogen-(C₁-C₄)-alkyl, (C₁-C₄)-Alkylthio, Halogen-(C₁-C₄)-alkylthio, (C₁-C₄)-Alkoxy und Halogen-(C₁-C₄)-alkoxy substituiertes (R¹¹)(C₁-C₄)-Alkylamino, (R¹¹)₂-Amino, R¹¹-Oxycarbonyl, R¹¹-Carbonyl, R¹¹-Carbonyloxy; (C₁-C₆)-Alkyl, (C₂-C₆)-Alkenyl, (C₂-C₆)-Alkinyl, (C₁-C₆)-Alkoxy-(C₁-C₆)-alkyl, (C₂-C₆)-Alkinyloxy-(C₁-C₆)-alkyl, (C₃-C₉)-Cycloalkyl, (C₃-C₉)-Cyloalkenyl, (C₁-C₆)-Alkoxy oder (C₁-C₆)-Alkylthio;
d) ein Rest der Formel Va, Vb, Vc, Vd, Vj oder Vp, oder
e) falls p für null steht, Oxo, NR⁸, N-OR⁸ oder N-NR⁸R⁹;
- R⁷: Wasserstoff, (C₁-C₄)-Alkylsulfonyl, Benzoyl oder Phenylsulfonyl, wobei die beiden letztgenannten Gruppen durch v Reste aus der Gruppe (C₁-C₂)-Alkyl, Halogen-(C₁-C₂)-alkyl, (C₁-C₂)-Alkoxy, Halogen-(C₁-C₂)-alkoxy, Halogen, Cyano und Nitro substituiert sind, und
- R¹¹: Wasserstoff, (C₁-C₄)-Alkyl, (C₂-C₄)-Alkenyl, (C₂-C₄)-Alkinyl oder (C₃-C₈)-Cycoalkyl bedeuten.

Bevorzugt sind Verbindungen der allgemeinen Formel I, worin
- x¹: die divalente Einheit O;
- R⁴: OR⁷, (C₁-C₄)-Alkylthio, (C₂-C₄)-Alkenylthio, (C₁-C₄)-Alkylsulfonyl, Cyano, Cyanato, Thiocyanato oder durch v Reste aus der Gruppe Halogen, (C₁-C₂)-Alkyl, (C₁-C₂)-Alkoxy, Halogen-(C₁-C₂)-alkyl, Halogen-(C₁-C₂)-alkoxy und Nitro substituiertes Phenylthio;
- R⁵: Wasserstoff, (C₁-C₄)-Alkyl, (C₃-C₈)-Cycloalkyl, (C₁-C₄)-Alkoxy, (C₁-C₄)-Alkylthio, Phenyl, oder zwei an direkt benachbarten Kohlenstoffatomen gebundene Reste R⁵ bilden mit den sie tragenden Kohlenstoffatomen einen substituierten 3- bis 6-gliedrigen Ring;
- R¹²: Wasserstoff, (C₁-C₄)-Alkyl, (C₂-C₄)-Alkenyl, oder sofern R¹¹ und R¹² an einem Atom oder an zwei direkt benachbarten Atomen gebunden sind, bilden sie gemeinsam mit den sie bindenden Atomen einen gesättigten, teilweise oder vollständig ungesättigten fünf- bis sechsgliedrigen Ring, der p Heteroatome aus der Gruppe Sauerstoff, Stickstoff und Schwefel enthält;
- Y: eine divalente Einheit aus der Gruppe CHR⁵ und C(R⁵)₂, und
- Z: eine divalente Einheit aus der Gruppe O, S, SO₂, N-(C₁-C₄)-Alkyl, CHR⁵ und C(R⁵)₂ bedeuten.

Ebenfalls bevorzugt sind Verbindungen der allgemeinen Formel 1, worin
- R²: Halogen, Halogen-(C₁-C₂)-alkyl oder (C₁-C₂)-Alkylsulfonyl;
- R⁵: (C₁-C₄)-Alkyl, (C₃-C₈)-Cycloalkyl, (C₁-C₄)-Alkoxy, (C₁-C₄)-Alkylthio, Phenyl, oder zwei an direkt benachbarten Kohlenstoffatomen gebundene Reste R⁵ bilden mit den sie tragenden Kohlenstoffatomen einen substituierten 3- bis 6-gliedrigen Ring;
- R⁷: Wasserstoff, (C₁-C₄)-Alkylsulfonyl, Benzoyl oder Phenylsulfonyl, und
- R⁸: Wasserstoff, Methyl oder Ethyl bedeuten, und
- R²: in 4-Position des Phenylrings steht.

Besonders bevorzugt sind Verbindungen der allgemeinen Formel 1, worin
- x²: eine geradkettige oder verzweigte (C₁-C₄)-Alkylen-, (C₂-C₄)-Alkenylen- oder (C₂-C₄)-Alkinylenkette;
- R¹: Chlor, Brom, Methyl, Trifluormethyl, Cyano oder Nitro;
- R²: Chlor, Brom, Methylsulfonyl, Ethylsulfonyl, Trifluormethyl oder Nitro;
- R⁴: OR⁷, (C₁-C₄)-Alkylthio, (C₂-C₄)-Alkenylthio oder Phenylthio;
- R⁵: Wasserstoff, (C₁-C₄)-Alkyl, oder zwei an direkt benachbarten Kohlenstoffatomen gebundene Reste R⁵ bilden mit den sie tragenden Kohlenstoffatomen einen substituierten 3- bis 6-gliedrigen Ring;
- A: eine divalente Einheit aus der Gruppe O, S(O)ₙ, NH und N-(C₁-C₆)-Alkyl;
- M: (C₁-C₆)-Alkylen;
- Y und Z: unabhängig voneinander eine divalente Einheit aus der Gruppe, CHR⁵ und C(R⁵)₂ bedeuten.

Die erfindungsgemäßen Verbindungen können je nach Bedeutung der Substituenten beispielsweise nach einem oder mehreren der in den folgenden Schemata angegebenen Verfahren hergestellt werden.

Durch die in Schema 1 angegebene Umsetzung eines Cyclohexandions der Formel (II) mit einem Benzoylderivat der Formel (III), worin T für Halogen, Hydroxy oder Alkoxy steht, können erfindungsgemäße Verbindungen der Formel (I) gemäß an sich bekannten Verfahren hergestellt werden. Solche Verfahren sind beispielsweise aus EP-A 0 90 062 und EP-B 0 186 117 bekannt.

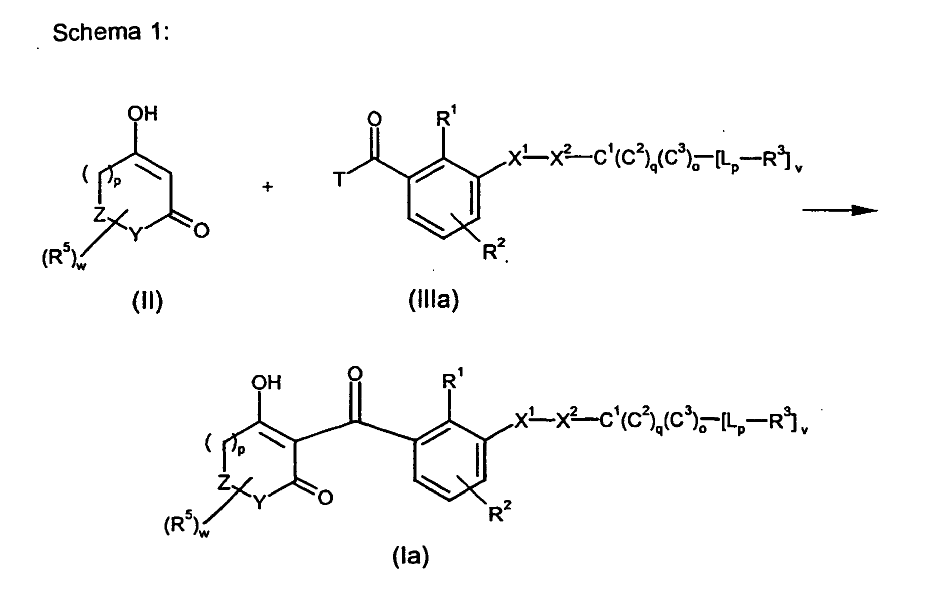

Verbindungen oben genannter Formel (IIIa) können aus Verbindungen der Formeln (IIIb) und (IVa), in der L' für eine Fluchtgruppe, wie Halogen, Mesyl, Tosyl und Triflat steht, gemäß an sich bekannten Methoden hergestellt werden. Solche Methoden sind beispielsweise aus Houben-Weyl Band 6/3, S. 54 bis 69, Band 9, S. 103 bis 115 und Band 11/1, S. 97 bekannt.

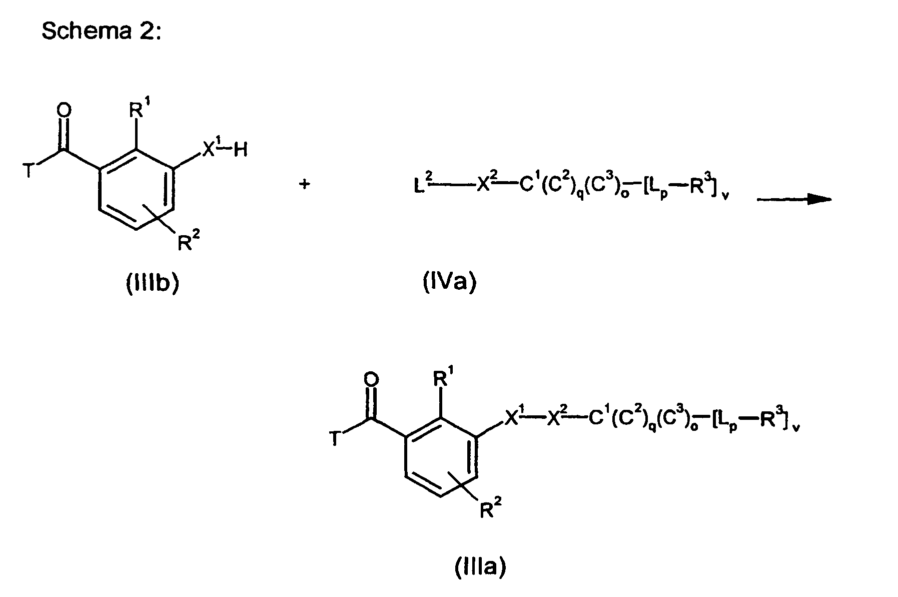

Verbindungen der Formel (IIIa) können auch beispielsweise gemäß der in Schema 3 beschriebenen Methode aus Verbindungen der Formeln (IIIc) und (IVb) hergestellt werden. Solche Methoden sind aus WO 98/42648, Houben-Weyl Band 6/3, S. 75 bis 78, Band 9, S. 103 bis 105 bekannt.

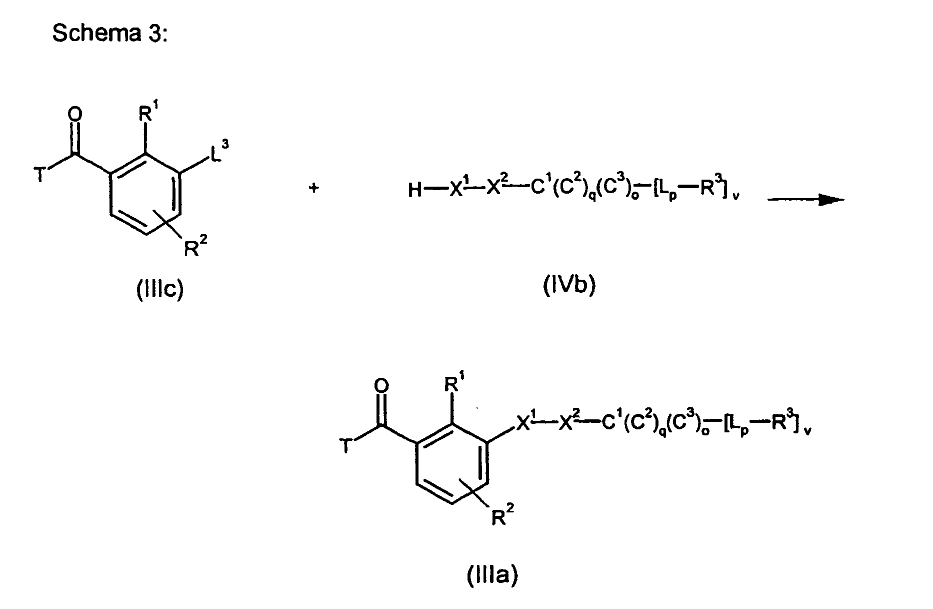

Erfindungsgemäße Verbindungen der Formel (I), in der R⁴ für andere Reste als Hydroxy steht, können beispielsweise gemäß Schema 4 hergestellt werden. Die darin angegebene Umsetzung einer Verbindung der Formel (la) mit einem Halogenierungsreagenz, wie Oxalylchlorid oder Oxalylbromid, führt zu erfindungsgemäßen Verbindungen der Formel (Ib), die durch Reaktion, gegebenenfalls unter Basenkatalyse, mit Nukleophilen, wie Alkalimetallcyaniden, Alkalimetallcyanaten, Alkalimetallthiocyanaten, Alkylthioalkoholen und Thiophenolen zu weiteren erfindungsgemäßen Verbindungen der Formel (Ic), in der R⁴ für Alkylthio, Halogenalkylthio, Alkenylthio, Halogenalkenylthio, Alkinylthio, Halogenalkinylthio, gegebenenfalls substituiertes Phenylthio, Cyano, Cyanato, Thiocyanato oder OR⁷ steht, umgesetzt werden können. Solche Reaktionen sind beispielsweise beschrieben in *Synthesis* 12, 1287 (1992). Durch Reaktion mit einem Oxidationsreagenz, wie m-Chlorperoxybenzoesäure, Peroxyessigsäure, Wasserstoffperoxid und Kaliumperoxymonosulfat, werden erfindungsgemäße Verbindungen der Formel (Ic) erhalten, in der R⁴ für Alkylsulfinyl, Halogenalkylsulfinyl, Alkenylsulfinyl, Halogenalkenylsulfinyl, Alkinylsulfinyl, Halogenalkinylsulfinyl, Alkylsulfonyl, Halogenalkylsulfonyl, Alkenylsulfonyl, Halogenalkenylsulfonyl, Alkinylsulfonyl, gegebenenfalls substituiertes Phenylthio oder Halogenalkinylsulfonyl steht. Solche Reaktionen sind beispielsweise beschrieben in J. Org. *Chem.* **53**, 532 (1988), *Tetrahedron* Lett. **21**, 1287 (1981).

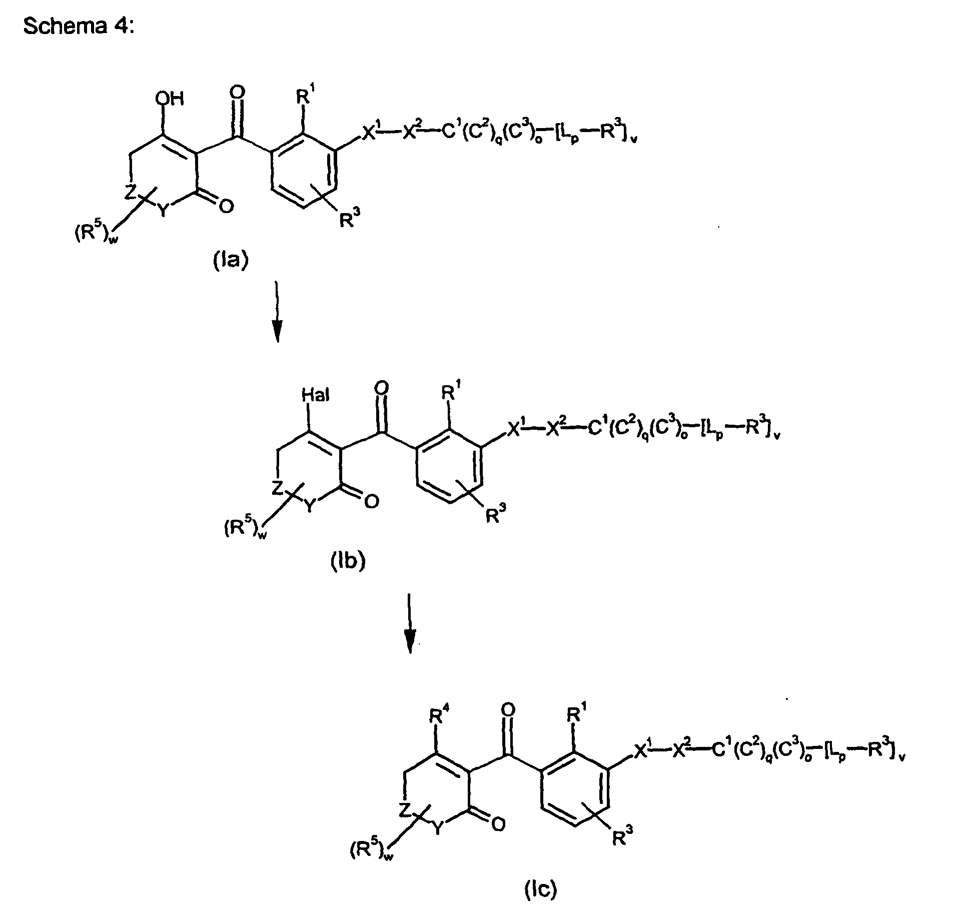

Verbindungen der Formeln (IIIa), mit Ausnahme der Verbindungen, in denen C¹ für Oxiranyl oder Oxetanyl und die Laufzahlen o und q beide null bedeuten, sind neu und ebenfalls Gegenstand der Erfindung.

Die erfindungsgemäßen Verbindungen der Formel (I) weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es in der Regel unerheblich, ob die Substanzen im Vorsaat-, Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll. Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, lmperata sowie Sorghum und auch ausdauemde Cyperusarten gut erfaßt. Bei dikotylen Unkrautarten erstreckt sich das Wirkungsspektrum auf Arten wie z.B. Galium, Viola, Veronica, Lamium, Stellaria, Amaranthus, Sinapis, Ipomoea, Sida, Matricaria und Abutilon auf der annuellen Seite sowie Convolvulus, Cirsium, Rumex und Artemisia bei den perennierenden Unkräutern. Unter den spezifischen Kulturbedingungen im Reis vorkommende Schadpflanzen wie z.B. Echinochloa, Sagittaria, Alisma, Eleocharis, Scirpus und Cyperus werden von den erfindungsgemäßen Wirkstoffen ebenfalls hervorragend bekämpft. Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab. Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen oder in Zierpflanzungen.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregem von Pflanzenkrankheiten wie bestimmten Insekten oder Mikroorganismen wie Pilzen, Bakterien oder Viren. Andere besondere Eigenschaften betreffen z. B. das Emtegut hinsichtlich Menge, Qualität, Lagerfähigkeit, Zusammensetzung und spezieller lnhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt ist die Anwendung der erfindungsgemäßen Verbindungen der Formel (1) oder deren Salze in wirtschaftlich bedeutenden transgenen Kulturen von Nutz-und Zierpflanzen, z. B. von Getreide wie Weizen, Gerste, Roggen, Hafer, Hirse, Reis und Mais oder auch Kulturen von Moniok, Zuckerrübe, Baumwolle, Soja, Raps, Kartoffel, Tomate, Erbse und anderen Gemüsesorten. Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylhamstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).

Zahlreiche molekularbiologische Techniken, mit denen neue transgene Pflanzen mit veränderten Eigenschaften hergestellt werden können, sind im Prinzip bekannt; siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996 oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Seqüenzen erlauben. Mit Hilfe der obengenannten Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106).

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h. sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen in transgenen Kulturen eingesetzt werden, welche gegen Herbizide aus der Gruppe der Sulfonylhamstoffe, Glufosinate-ammonium oder Glyphosate-isopropylammonium und analoge. Wirkstoffe resistent sind.

Bei der Anwendung der efindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen. Gegenstand der Erfindung ist deshalb auch die Verwendung der erfindungsgemäßen Verbindungen als Herbizide zur Bekämpfung von Schadpflanzen in transgenen Kulturpflanzen.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsef ein und können damit zur gezielten Beeinflussung von Pflanzeninhaltsstoffen und zur Emteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) enthalten.

Die Verbindungen der Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasserin-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, 2,2'-dinaphthylmethan-6,6'-disulfonsaures Natrium, ligninsulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Ernulsionen (EW), lassen sich beispielsweise mittels Rührern, Kolloidmühlen und/oder statischen Mischem unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbeibett-Granullerung, Teller-Granulierung, Mischung mit Hochgeschwindigkeitsmischem und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I). In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Fomnulienrngen lassen sich auch Kombinationen mit anderen pestizid wirksamen Stoffen, wie z.B. Insektiziden, Akariziden, Herbiziden, Fungiziden, sowie mit Safenem, Düngemitteln und/oder Wachstumsregulatoren herstellen, z.B. in Form einer Fertigformulierung oder als Tankmix.

Als Kombinationspartner für die erfindungsgemäßen Wirkstoffe in Mischungsformulierungen oder im Tank-Mix sind beispielsweise bekannte Wirkstoffe einsetzbar, wie sie z.B. in Weed Research 26, 441-445 (1986) oder "The Pesticide Manual", 11th edition, The British Crop Protection Council and the Royal Soc. of Chemistry, 1997 und dort zitierter Literatur beschrieben sind. Als bekannte Herbizide, die mit den Verbindungen der Formel (I) kombiniert werden können, sind z.B. folgende Wirkstoffe zu nennen (Anmerkung: Die Verbindungen sind entweder mit dem "common name" nach der international Organization for Standardization (ISO) oder mit dem chemischen Namen, ggf. zusammen mit einer üblichen Codenummer bezeichnet):
acetochlor; acifluorfen; aclonifen; AKH 7088, d.h. [[[1-[5-[2-Chloro-4-(trifluoromethyl)-phenoxy]-2-nitrophenyl]-2-methoxyethylidene]-amino]-oxy]-essigsäure und -essigsäuremethylester; alachlor; alloxydim; ametryn; amidosulfuron; amitrol; AMS, d.h. Ammoniumsulfarnat; anilofos; asulam; atrazin; azimsulfurone (DPX-A8947); aziprotryn; barban; BAS 516 H, d.h. 5-Fluor-2-phenyl-4H-3,1-benzoxazin-4-on; benazolin; benfluralin; benfuresate; bensulfuron-methyl; bensulide; bentazone; benzofenap; benzofluor; benzoylprop-ethyl; benzthiazuron; bialaphos; bifenox; bromacil; bromobutide; bromofenoxim; bromoxynil; bromuron; buminafos; busoxinone; butachlor; butamifos; butenachlor; buthidazole; butralin; butylate; cafenstrole (CH-900); carbetamide; cafentrazone (ICI-A0051); CDAA, d.h. 2-Chlor-N,N-di-2-propenylacetamid; CDEC, d.h. Diethyldithiocarbaminsäure-2-chlorallylester; chlomethoxyfen; chloramben; chlorazifop-butyl, chlormesulon (ICI-A0051); chlorbromuron; chlorbufam; chlorfenac; chlorflurecol-methyl; chloridazon; chlorimuron ethyl; chlomitrofen; chlorotoluron; chloroxuron; chlorpropham; chlorsulfuron; chlorthal-dimethyl; chlorthiamid; cinmethylin; cinosutfuron; clethodim; clodinafop und dessen Esterderivate (z.B. clodinafop-propargyl); clomazone; clomeprop; cloproxydim; clopyralid; cumyluron (JC 940); cyanazine; cycloate; cyclosulfamuron (AC 104); cycloxydim; cycluron; cyhalofop und dessen Esterderivate (z.B. Butylester, DEH-112); cyperquat; cyprazine; cyprazole; daimuron; 2,4-DB; dalapon; desmedipham; desmetryn; di-allate; dicamba; dichlobenil; dichlorprop; diclofop und dessen Ester wie diclofop-methyl; diethatyl; difenoxuron; difenzoquat; diflufenican; dimefuron; dimethachlor; dimethametryn; dimethenamid (SAN-582H); dimethazone, clomazon; dimethipin; dimetrasulfuron, dinitramine; dinoseb; dinoterb; diphenamid; dipropetryn; diquat; dithiopyr, diuron; DNOC; eglinazine-ethyl; EL 77, d.h. 5-Cyano-1-(1,1-dimethylethyl)-N-methyl-1H-pyrazole-4-carboxamid; endothal; EPTC; esprocarb; ethalfluralin; ethametsulfuron-methyl; ethidimuron; ethiozin; ethofumesate; F5231, d.h. N-[2-Chlor-4-fluor-5-[4-(3-fluorpropyl)-4,5-dihydro-5-oxo-1H-tetrazol-1-yl]-phenyl]-ethansulfonamid; ethoxyfen und dessen Ester (z.B. Ethylester, HN-252); etobenzanid (HW 52); fenoprop; fenoxan, fenoxaprop und fenoxaprop-P sowie deren Ester, z.B. fenoxaprop-P-ethyl und fenoxaprop-ethyl; fenoxydim; fenuron; flamprop-methyl; flazasulfuron; fluazifop und fluazifop-P und deren Ester, z.B. fluazifop-butyl und fluazifop-P-butyl; fluchloralin; flumetsulam; flumeturon; flumiclorac und dessen Ester (z.B. Pentylester, S-23031); flumioxazin (S-482); flumipropyn; flupoxam (KNW-739); fluorodifen; fluoroglycofen-ethyl; flupropacil (UBIC-4243); fluridone; flurochloridone; fluroxypyr; flurtamone; fomesafen; fosamine; furyloxyfen; glufosinate; glyphosate; halosafen; halosulfuron und dessen Ester (z.B. Methylester, NC-319); haloxyfop und dessen Ester; haloxyfop-P (= R-haloxyfop) und dessen Ester, hexazinone; imazapyr; imazamethabenz-methyl; imazaquin und Salze wie das Ammoniumsalz; ioxynil; imazethamethapyr; imazethapyr; imazosulfuron; isocarbamid; isopropalin; isoproturon; isouron; isoxaben; isoxapyrifop; karbutilate; lactofen; lenacil; linuron; MCPA; MCPB; mecoprop; mefenacet; mefluidid; metamitron; metazachlor, metham; methabenzthiazuron; methazole; methoxyphenone; methyldymron; metabenzuron, methobenzuron; metobromuron; metolachlor, metosulam (XRD 511); metoxuron; metribuzin; metsulfuron-methyl; MH; molinate; monalide; monolinuron; monuron; monocarbamide dihydrogensulfate; MT 128, d.h. 6-Chlor-N-(3-chlor-2-propenyl)-5-methyl-N-phenyl-3-pyridazinamin; MT 5950, d.h. N-[3-Chlor-4-(1-methylethyl)-phenyl]-2-methylpentanamid; naproanilide; napropamide; naptalam; NC 310, d.h. 4-(2,4-dichlorbenzoyl)-1-methyl-5-benzyloxypyrazol; neburon; nicosulfuron; nipyraclophen; nitralin; nitrofen; nitrofluorfen; norflurazon; orbencarb; oryzalin; oxadiargyl (RP-020630); oxadiazon; oxyfluorfen; paraquat; pebulate; pendimethalin; perfluidone; phenisopham; phenmedipham; picloram; piperophos; piributicarb; pirifenop-butyl; pretilachlor; primisulfuron-methyl; procyazine; prodiamine; profluralin; proglinazine-ethyl; prometon; prometryn; propachlor, propanil; propaquizafop und dessen Ester; propazine; propham; propisochlor; propyzamide; prosulfalin; prosulfocarb; prosulfuron (CGA-152005); prynachlor, pyrazolinate; pyrazon; pyrazosulfuron-ethyl; pyrazoxyfen; pyridate; pyrithiobac (KIH-2031); pyroxofop und dessen Ester (z.B. Propargylester); quinclorac; quinmerac; quinofop und dessen Esterderivate, quizalofop und quizalofop-P und deren Esterderivate z.B. quizalofop-ethyl; quizalofop-P-tefuryl und -ethyl; renriduron; rimsulfuron (DPX-E 9636); S 275, d.h. 2-[4-Chlor-2-fluor-5-(2-propynyloxy)-phenyl]-4,5,6,7-tetrahydro-2H-indazol; secbumeton; sethoxydim; siduron; simazine; simetryn; SN 106279, d.h. 2-[[7-[2-Chlor-4-(trifluor-methyl)-phenoxy]-2-naphthalenyl]-oxy]-propansäure und - methylester, sulfentrazon (FMC-97285, F-6285); sulfazuron; sulfometuron-methyl; sulfosate (ICI-A0224); TCA; tebutam (GCP-5544); tebuthiuron; terbacil; terbucarb; terbuchlor; terbumeton; terbuthylazine; terbutryn; TFH 450, d.h. N,N-Diethyl-3-[(2-ethyl-6-methylphenyl)-sulfonyl]-1H-1,2,4-triazol-1-carboxamid; thenylchlor (NSK-850); thiazafluron; thiazopyr (Mon-13200); thidiazimin (SN-24085); thiobencarb; thifensulfuron-methyl; tiocarbazil; tralkoxydim; tri-allate; triasulfuron; triazofenamide; tribenuron-methyl; triclopyr; tridiphane; trietazine; trifluralin; triflusulfuron und Ester (z.B. Methylester, DPX-66037); trimeturon; tsitodef; vemolate; WL 110547, d.h. 5-Phenoxy-1-[3-(trifluormethyl)-phenyl]-1 H-tetrazol; UBH-509; D-489; LS 82-556; KPP-300; NC-324; NC-330; KH-218; DPX-N8189; SC-0774; DOWCO-535; DK-8910; V-53482; PP-600; MBH-001; KIH-9201; ET-751; KIH-6127 und KIH-2023.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 10,0 kg/ha oder mehr Aktivsubstanz, vorzugsweise liegt sie jedoch zwischen 0,005 und 5 kg/ha.

Die nachstehenden Beispiele erläutern die Erfindung.

### A. Chemische Beispiele

### 1.1 Herstellung von 2-(2-Chlor-3-cyclopentylmethoxy-4-ethylsulfonyl-benzoyl)-cyclohexan-1,3-dion

Die als Ausgangsmaterial verwendete Verbindung 2-Chlor-3-hydroxy-4-ethylsulfonyl-benzoesäure wurde gemäß der in EP-A 0 195 247 beschriebenen Methode hergestellt. 2,4-Dibrom-3-hydroxy-benzoesäureethylester wurde gemäß der in US 5,026,896 beschriebenen Methode hergestellt. Die Herstellung von Methansulfonsäure-cyclopentylcarbinylester erfolgte gemäß J. Org. Chem 45, 9 (1980) 1707-1708.

Schritt 1: 2-Chlor-3-hydroxy-4-ethylsulfonyl-benzoesäuremethylester 33.0 g (124.7 mmol) 2-Chlor-3-hydroxy-4-ethylsulfonyl-benzoesäure wurden in 1300 ml Methanol gelöst. Es wurden 174 ml (3263 mmol) konz. H₂SO₄ zugetropft, und die Mischung wurde 5 h unter Rückfuß erhitzt. Das Reaktionsgemisch wurde am Rotationsverdampfer eingeengt, und der Rückstand wurde in Methylenchlorid aufgenommen. Es wurde mit Waser gewaschen, über Na₂SO₄ getrocknet und am Rotationsverdampfer vollständig eingeengt. Man erhielt 2-Chlor-3-hydroxy-4-ethylsulfonyl-benzoesäuremethylester als gelbes, zähes Öl.

| | | |
|---|---|---|
| Ausbeute: | 28.23 g (81 % der Theorie) | Rf (Essigester) 0.45 |
| ¹H-NMR: | δ [CDCl₃] 1.32 (t, 3H), 3.24 (q, 2H), 3.96 (s, 3H), 7.38 (d, 1H), 7.65 (d, 1H) | |

Schritt 2: 2-Chlor-3-cyclopentylmethoxy-4-ethylsulfonyl-benzoesäuremethyl-ester 1.488 g (10.8 mmol) Kaliumcarbonat und 1.343 g (7.5 mmol) Methansulfonsäure-cyclopentylcarbinylester wurden in 30 ml N,N-Dimethylformamid vorgelegt. Bei Raumtemperatur wurden 1.50 g (5.4 Mol) 2-Chlor-3-hydroxy-4-ethylsulfonyl-benzoesäuremethylester zugegeben, und die Mischung wurde für 5 h auf 70-80°C erhitzt. Anschliessend wurde auf Wasser gegeben und mit Diethylether extrahiert. Die vereinigten organischen Phasen wurden mit Wasser gewaschen, über Na₂SO₄ getrocknet und am Rotationsverdamper vollständig eingeengt. Trocknen im Ölpumpenvakuum ergab 2-Chlor-3-cyclopentylmethoxy-4-ethylsulfonyl-benzoesäuremethylester als braunes Öl.

| | | |
|---|---|---|
| Ausbeute: | 1.10 g (56% der Theorie) | Rf (Essigester) 0.77 |
| ¹H-NMR: | δ [CDCl₃] 1.23 (t, 3H), 1.43 (m, 2H), 1.63 (m, 4H),1.84 (m, 2H), 2.51 (m, 1 H), 3.43 (q, 2H), 3.95 (s, 3H), 4.13 (d, 2H), 7.60 (d, 1 H), 7.89 (d, 1H) | |

Schritt 3: 2-Chlor-3-cyclopentylmethoxy-4-ethylsulfonyl-benzoesäure 1.100 g (3.00 mmol) 2-Chlor-3-cyclopentylmethoxy-4-ethylsulfonyl-benzoesäure-methylester wurden in einer Mischung aus 20 ml Tetrahydrofuran und 20 ml Wasser gelöst und mit 0.134 g (3.40 mmol) Natriumhydroxid versetzt. Die Mischung wurde 12 h bei Raumtemperatur gerührt und am Rotationsverdampfer vollständig eingeengt. Der Rückstand wurde in Wasser aufgenommen und mit 6 N HCl versetzt. Die erhaltene Mischung wurde zweimal mit Methylenchlorid extrahiert, über Na₂SO₄ getrocknet und am Roationsverdampfer vollständig eingeengt. Es wurde 2-Chlor-3-(cyclopentyl-methoxy)-4-ethylsulfonyl-benzoesäure in Form eines zähen Öls erhalten.

| | | |
|---|---|---|
| Ausbeute: | 1.04 g (100% der Theorie) | Rf (Essigester) 0.59 |
| ¹H-NMR: | δ [CDCl₃] 1.24 (t, 3H), 1.45 (m, 2H), 1.62 (m, 4H),1.84 (m, 2H), 2.52 (m, 1 H), 3.43 (q, 2H), 4.13 (d, 2H), 7.76 (d, 1H), 7.93 (d, 1H) | |

Schritt 4: 2-Chlor-3-cyclopentylmethoxy-4-ethylsulfonylbenzoesäure-3-oxo-1-cyclohexenylester
0.550 g (1.60 mmol) 2-Chlor-3-cyclopentylmethoxy-4-ethylsulfonyl-benzoesäure, 0.196 g (1.70 mmol) Cyclohexan-1,3-dion, 0.279 g (1.40 mmol) N'-(3-Dimethylaminopropyl)-N-ethylcarbodiimid Hydrochlorid und 0.002 g Dimethylaminopyridin wurden in 15 ml Methylenchlorid 10 h bei Raumtemperatur gerührt. Anschliessend wurde mit Methylenchlorid verdünnt und mit 0.5 N HCl, mit Wasser, mit gesättigter NaHCO₃-Lösung und wieder mit Wasser gewaschen. Nach Trocknen der vereinigten organischen Phasen über Na₂SO₄ und vollständigem Einengen am Rotationsverdampfer wurde 2-Chlor-3-cyclopentylmethoxy-4-ethylsulfonylbenzoesäure-3-oxo-1-cyclohexenylester in Form eines braunen Harzes erhalten.

| | | |
|---|---|---|
| Ausbeute: | 0.335 g (47% der Theorie) | Rf (Essigester): 0.68 |
| ¹H-NMR: | δ [CDCl₃] 1.23 (t, 3H), 1.44 (m, 2H), 1.64 (m, 4H), 1.85 (m, 2H), 2.15 (m, 2H), 2.47 (m, 2H), 2.53 (m, 1H), 2.68 (m, 2H), 3.25 (q, 2H), 4.15 (d, 2H), 6.08 (s, 1 H), 7.71 (d, 1H), 7.96 (d, 1H) | |

Schritt 5: 2-(2-Chlor-3-cyclopentylmethoxy-4-ethylsulfonylbenzoyl)-cyclohexan-1,3-dion
0.290 g (0.70 mmol) (3-Oxo-1-cyclohexenyl)-2-chlor-3-(cyclopentyl-methoxy)-4-ethylsulfonyl)benzoat wurde in 10 ml Acetonitril gelöst. Es wurden 3 Tropfen Acetoncyanhydrin sowie 0.117 g (1.20 mmol) Triethylamin zugegeben. Die Mischung wurde 2·h bei Raumtemperatur gerührt, worauf hin 0.013 g (0.20 mmol) Kaliumcyanid zugegeben wurden. Nach weiteren 10 h bei Raumtempeatur wurde vollständig eingeengt, der Rückstand in Wasser aufgenommen und mit 6 N Salzsäure versetzt. Anschliessend wurde mit Methylenchlorid extrahiert. Nach Trocknen der vereinigten organischen Phasen über Na₂SO₄, vollständigem Einengen am Rotationsverdampfer und Chromatographie an reversed-phase-Kieselgel (Laufmittel: Acetonitril/Wasser-Gradient) erhielt man 2-(2-Chlor-3-cyclopentylmethoxy-4-ethylsulfonylbenzoyl)-cyclohexan-1,3-dion in Form eines farblosen, zähen Öls.

| | | |
|---|---|---|
| Ausbeute: | 0.175 g (57% der Theorie) | Rf (Essigester): 0.50 |
| ¹H-NMR: | δ [CDCl₃] 1.25 (t, 3H), 1.45 (m, 2H), 1.60 (m, 4H), 1.82 (m, 2H), 2.05 (m, 2H), 2.44 (m, 2H), 2.50 (m, 1H), 2.80 (m, 2H), 3.23 (q, 2H), 4.11 (d, 2H), 7.05 (d, 1 H), 7.90 (d, 1H) | |

Herstellung von 2-(2,4-Dibrom-3-cyclobutylmethoxybenzoyl)-cyclohexan-1,3-dion

Schritt 1: 2,4-Dibrom-3-cyclobutylmethoxy-benzoesäureethylester
2.990 g (21.60 mmol) Kaliumcarbonat und 3.050 g (9.40 mmol) 2,4-Dibrom-3-hydroxy-benzoesäureethylester wurden in 50 ml N,N-Dimethylformamid vorgelegt. Bei Raumtemperatur wurden 1.401 g (9.40 mmol) Brommethylcyclobutan zugegeben, und die Mischung wurde für 6 h auf 120-130°C erhitzt. Anschliessend wurde auf Wasser gegeben und mit Diethylether extrahiert. Die vereinigten organischen Phasen wurden mit Wasser gewaschen, über Na₂SO₄ getrocknet und am Rotationsverdamper vollständig eingeengt. Trocknen im Ölpumpenvakuum ergab 2,4-Dibrom-3-cyclobutylmethoxy-benzoesäureethylester als braunes Öl.

| | | |
|---|---|---|
| Ausbeute: | 3.10 g (85% der Theorie) | Rf (Essigester) 0.88 |
| ¹H-NMR: | δ [CDCl₃] 1.20 (t, 3H), 1.87-2.26 (m, 6H), 2.87 (m, 1H), 4.00 (d, 2H), 4.19 (q, 2H), 7.31 (d, 1 H), 7.54 (d, 1H) | |

Schritt 2: 2,4-Dibrom-3-cyclobutylmethoxy-benzoesäure
3.000 g (7.30 mmol) 2,4-Dibrom -3-(cyclobutyl-methoxy)-benzoesäureethylester wurden in einer Mischung aus 30 ml Tetrahydrofuran und 30 ml Wasser gelöst und mit 0.436 g (10.90 mmol) Natriumhydroxid versetzt. Die Mischung wurde 12 h bei Raumtemperatur gerührt und am Rotationsverdampfer vollständig eingeengt. Der Rückstand wurde in Wasser aufgenommen und mit 6 N HCl versetzt. Die erhaltene Mischung wurde zweimal mit Methylenchlorid extrahiert, über Na₂SO₄ getrocknet und am Roationsverdampfer vollständig eingeengt. Es wurde 2,4-Dibrom -3-cyclobutylmethoxy-benzoesäure in Form eines zähen Öls erhalten.

| | | |
|---|---|---|
| Ausbeute: | 2.50 g (94% der Theorie) | Rf (Essigester) 0.60 |
| ¹H-NMR: | δ [CDCl₃] 1.87-2.09 (m, 4H), 2.09-2.23 (m, 2H), 2.87 (m, 1H), 4.02 (d, 2H), 7.53 (d, 1 H), 7.59 (d, 1H) | |

Schritt 3: 2,4-Dibrom-3-cyclobutylmethoxy-benzoesäure-3-oxo-1-cyclohexenyl-ester
1.150 g (3.20 mmol) 2,4-Dibrom -3-cyclobutylmethoxy-benzoesäure, 0.39 g (3.50 mmol) Cyclohexan-1,3-dion, 0.618 g (3.20 mmol) N'-(3-Dimethylaminopropyl)-N-ethylcarbodiimid Hydrochlorid und 0.004 g Dimethylaminopyridin wurden in 30 ml Methylenchlorid 10 h bei Raumtemperatur gerührt. Anschliessend wurde mit Methylenchlorid verdünnt und mit 0.5 N HCl, mit Wasser, mit gesättigter NaHCO₃₋Lösung und wieder mit Wasser gewaschen. Nach Trocknen der vereinigten organischen Phasen über Na₂SO₄ und verständigem Einengen am Rotationsverdampfer wurde 2,4-Dibrom-3-cyclobutylmethoxy-benzoesäure-3-oxo-1-cyclohexenyl-ester in Form eines gelben Harzes erhalten.

| | | |
|---|---|---|
| Ausbeute: | 0.80 g (55% der Theorie) | Rf (Essigester): 0.88 |
| ¹H-NMR: | δ [CDCl₃] 1.88-2.23 (m, 8H), 2.45 (m, 2H), 2.68 (m, 2H), 2.87 (m, 1H), 4.15 (d, 2H), 6.05 (s, 1H). 7.44 (d, 1H), 7.60 (d, 1H) | |

Schritt 4: 2-(2,4-Dibrom-3-cyclobutylmethoxybenzoyl)-cyclohexan-1,3-dion
0.220 g (0.50 mmol) 2-(2,4-Dibrom-3-cyclobutylmethoxy-benzoesäure-3-oxo-1-cyclohexenyl-ester wurden in 15 ml Acetonitril gelöst. Es wurden 3 Tropfen Acetoncyanhydrin sowie 0.121 g (1.20 mmol) Triethylamin zugegeben. Die Mischung wurde 2 h bei Raumtemperatur gerührt, woraufhin 0.031 g (0.5 mmol) Kaliumcyanid zugegeben wurden. Nach weiteren 10 h bei Raumtempeatur wurde vollständig eingeengt, der Rückstand in Wasser aufgenommen und mit 6 N Salzsäure versetzt. Anschliessend wurde mit Methylenchlorid extrahiert. Nach Trocknen der vereinigten organischen Phasen über Na₂SO₄, vollständigem Einengen am Rotationsverdampfer und Chromatographie an Kieselgel (Laufmittel: Toluol/THF) erhielt man erhielt man 2-(2,4-Dibrom-3-cyclobutylmethoxybenzoyl)-cyclohexan-1,3-dion in Form eines farblosen Öls.

| | | |
|---|---|---|
| Ausbeute: | 0.10 g (44% der Theorie) | Rf (Essigester): 0.60 |
| ¹H-NMR: | δ [CDCl₃] 1.80-2.16 (m, 8H), 2.58 (m, 2H), 2.72 (m, 2H), 2.80 (m, 1H), 3.92 (d, 2H), 6.71 (d, 1H), 7.46 (d, 1H) | |

Die in nachfolgenden Tabellen aufgeführten Beispiele wurden analog oben genannten Methoden hergestellt beziehungsweise sind analog oben genannten Methoden erhältlich.

Die verwendeten Abkürzungen bedeuten:

| | | | | | |
|---|---|---|---|---|---|
| Me = | Methyl | Et = | Ethyl | Ph = | Phenyl |
| Ac = | Acetyl | Pr = | Propyl | t = | tertiär |
| i = | iso | c = | cyclo | Fp. = | Festpunkt |
| R_{f} = | Retentionswert | | | | |

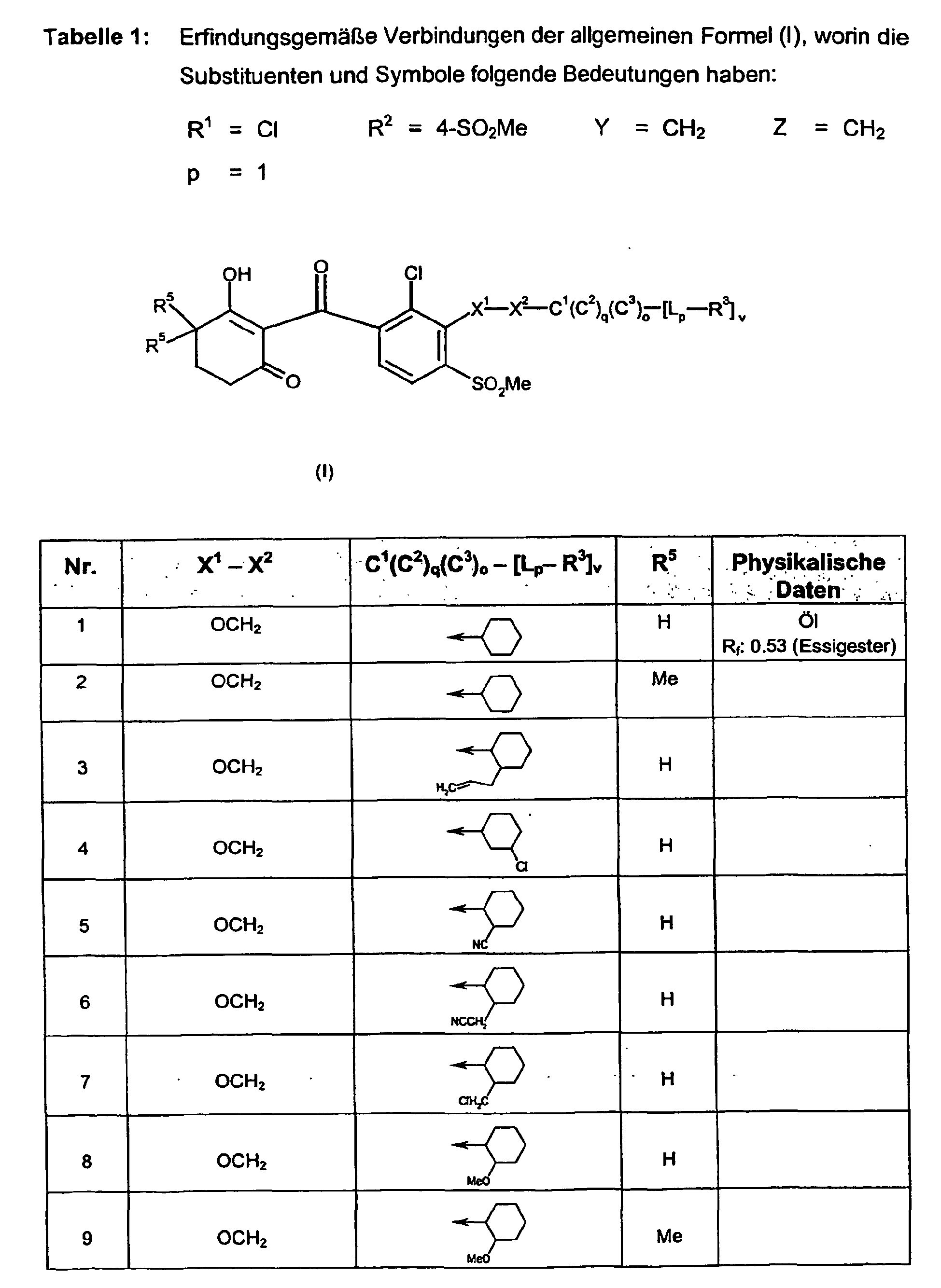

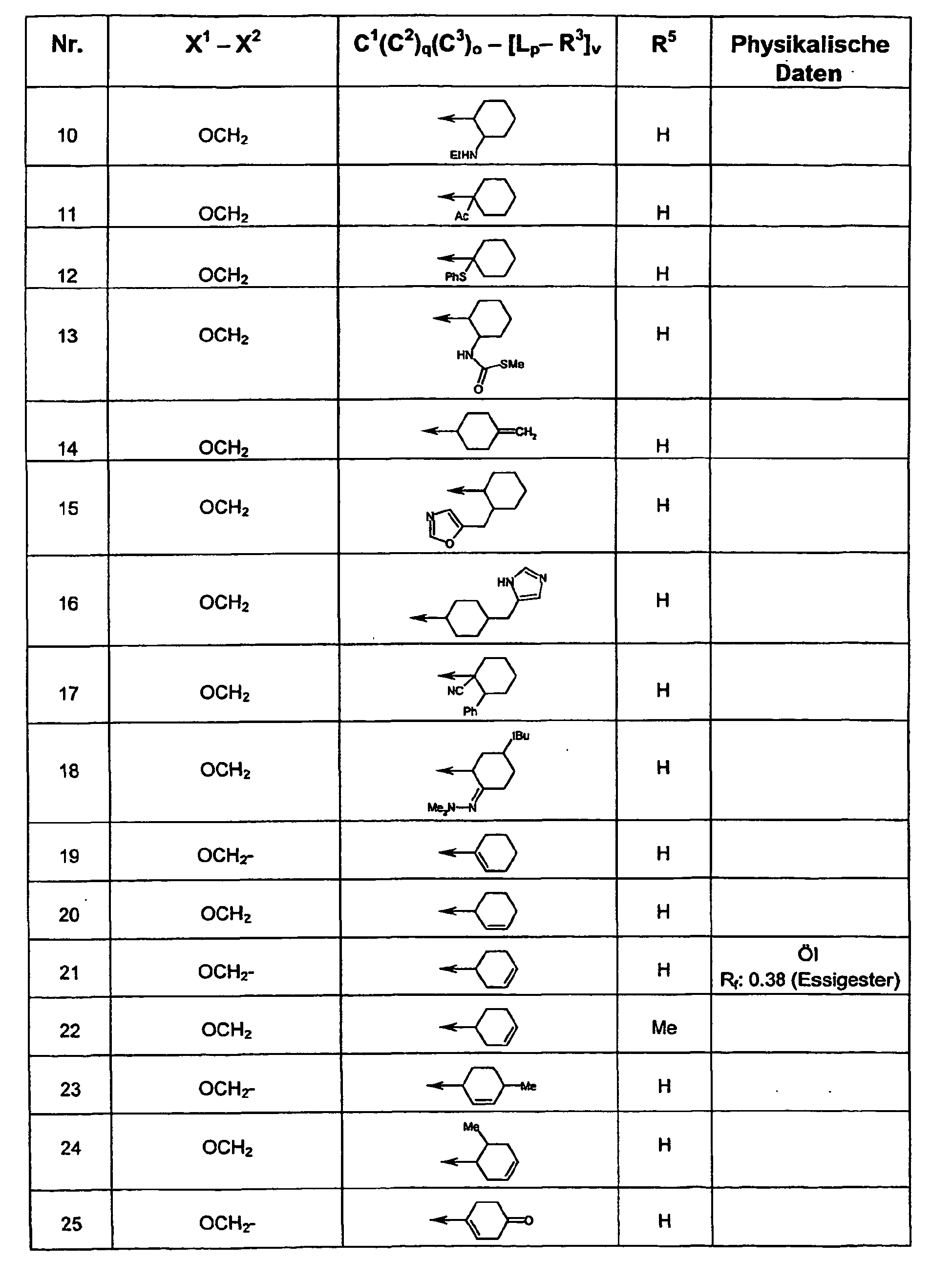

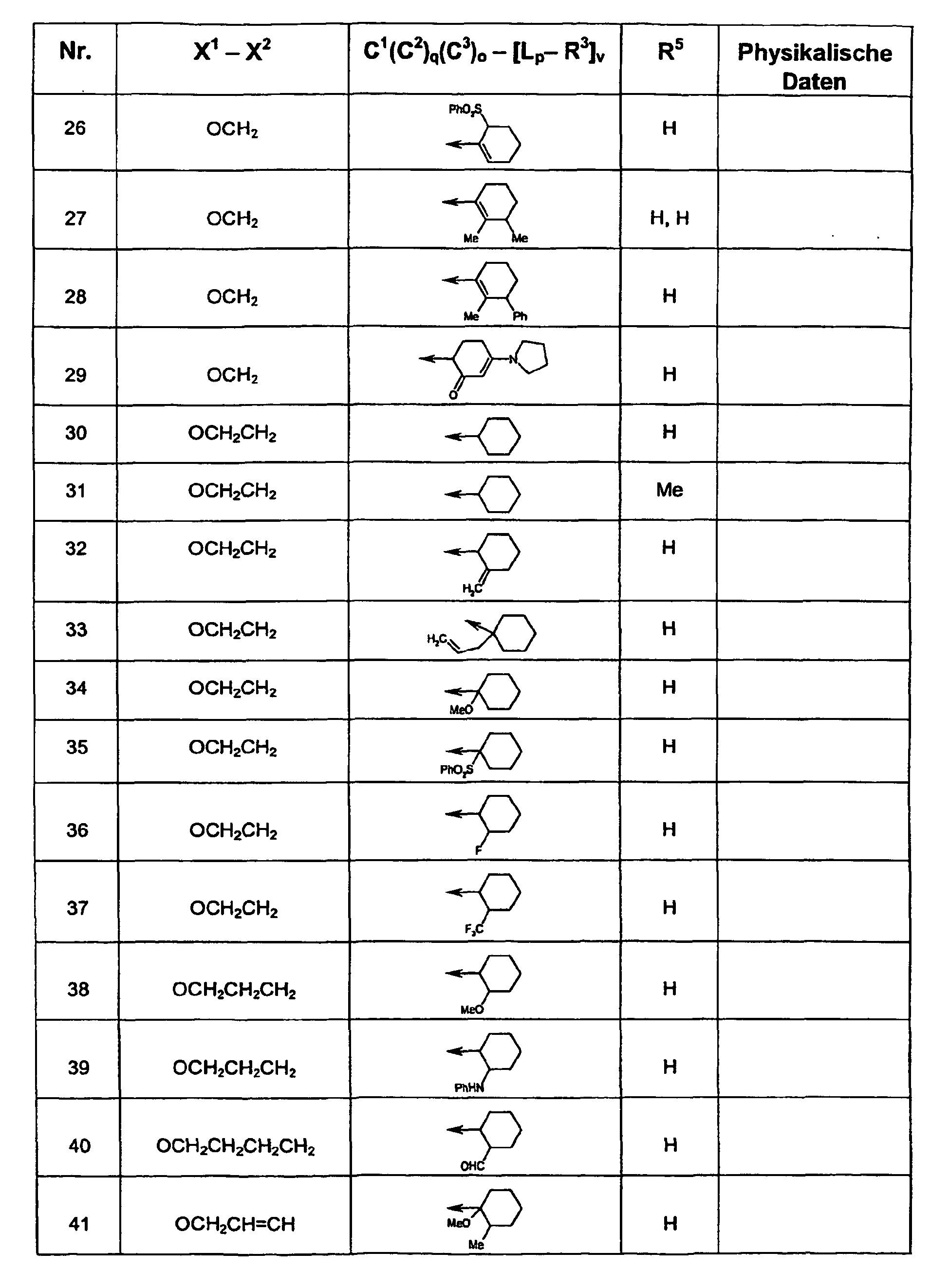

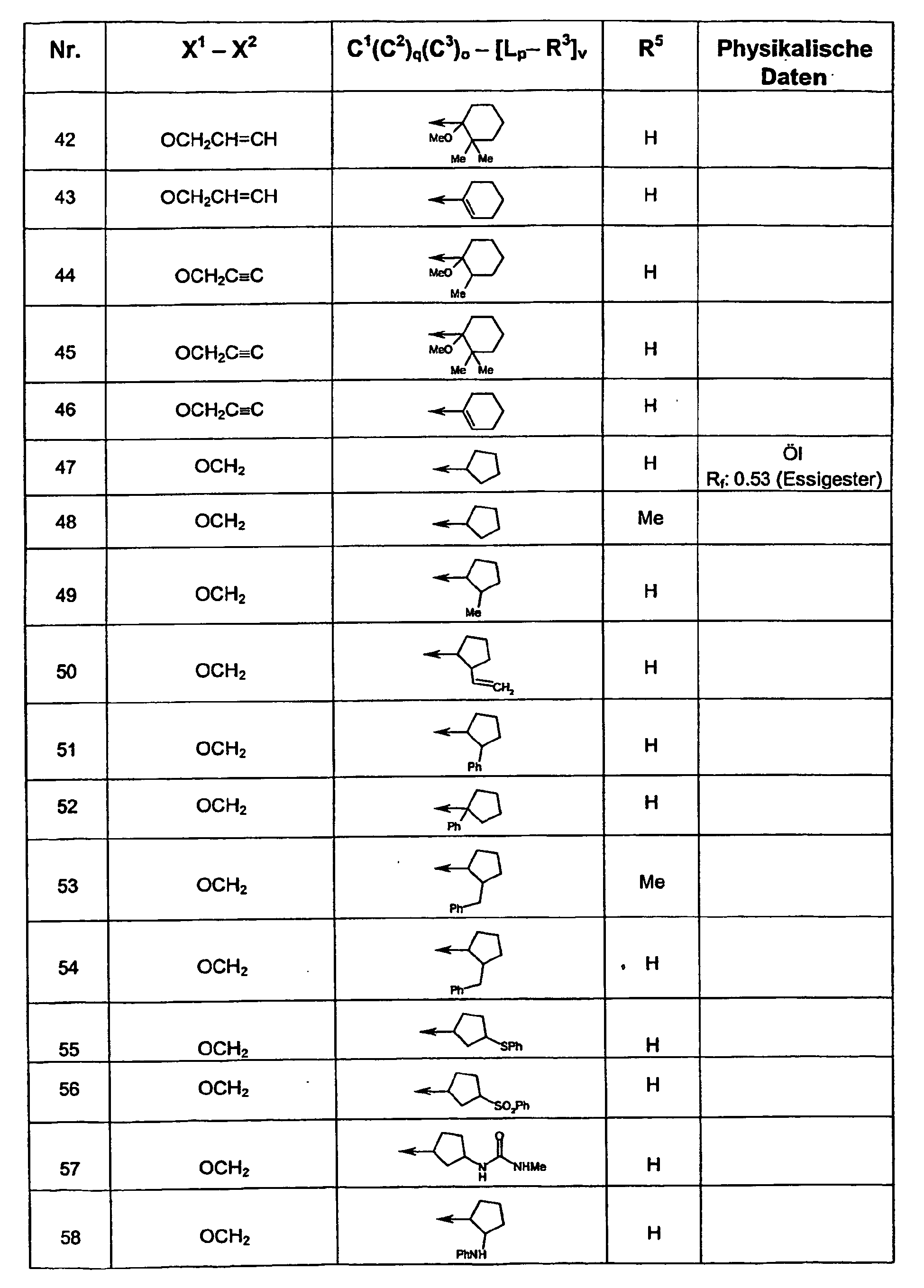

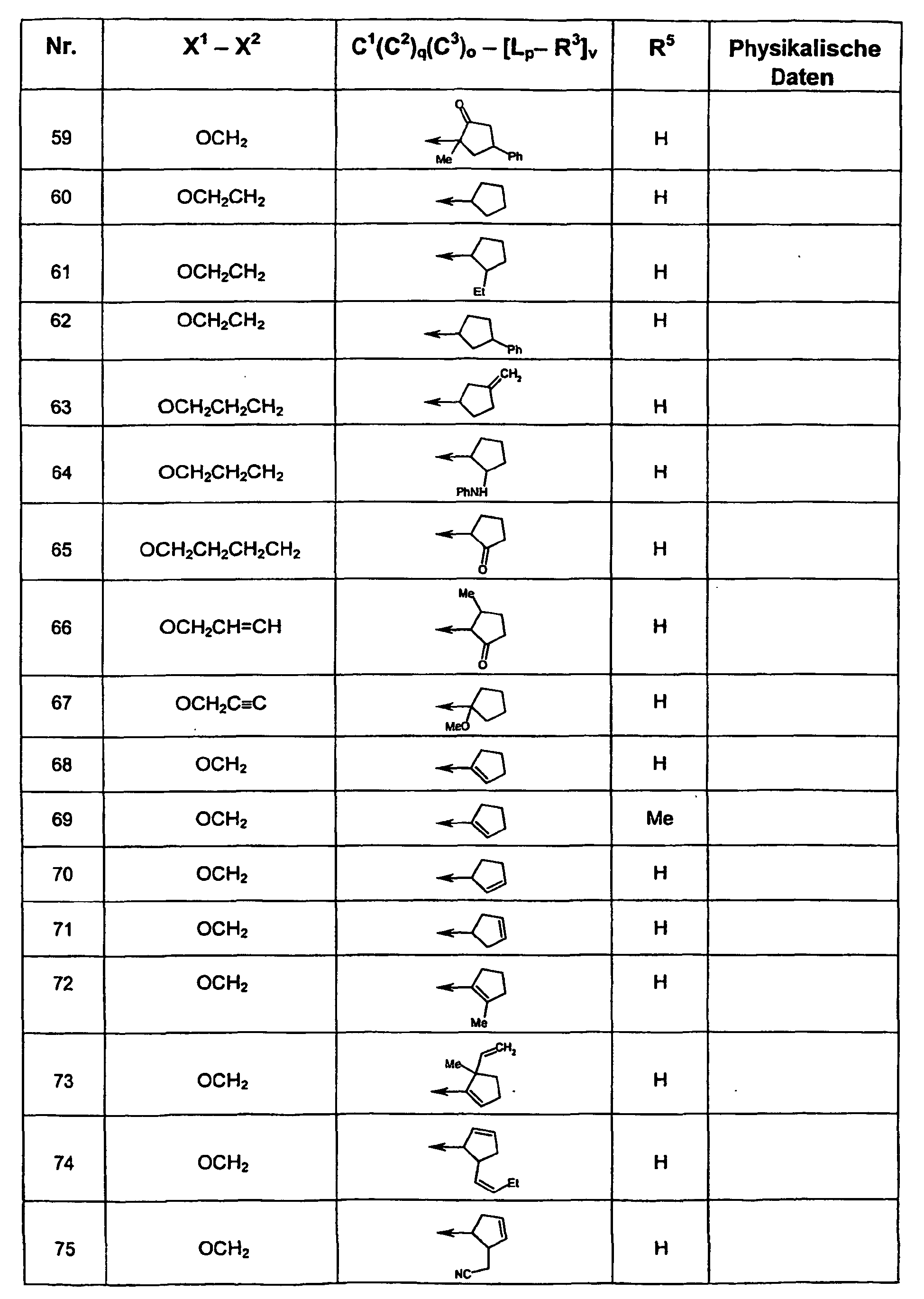

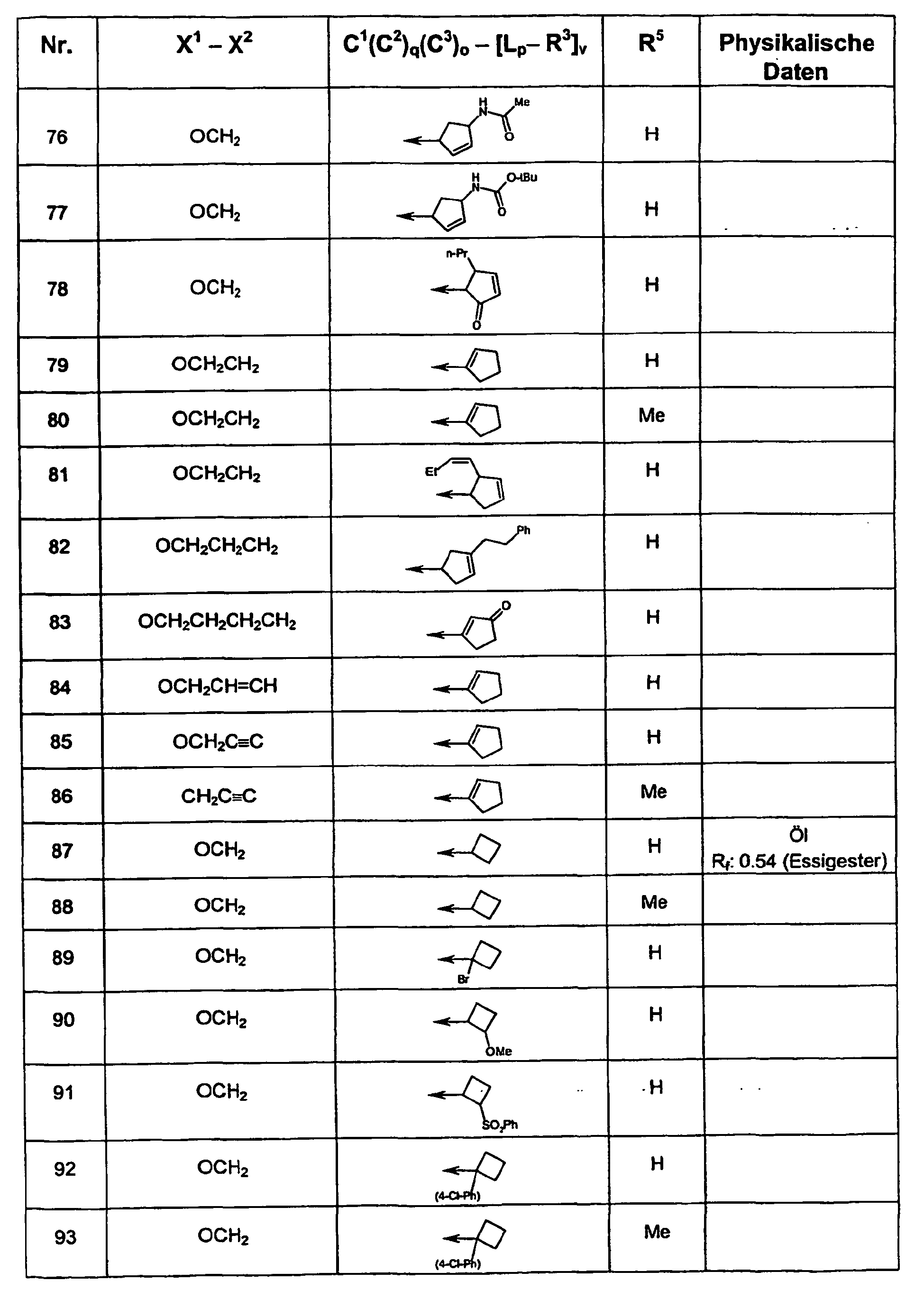

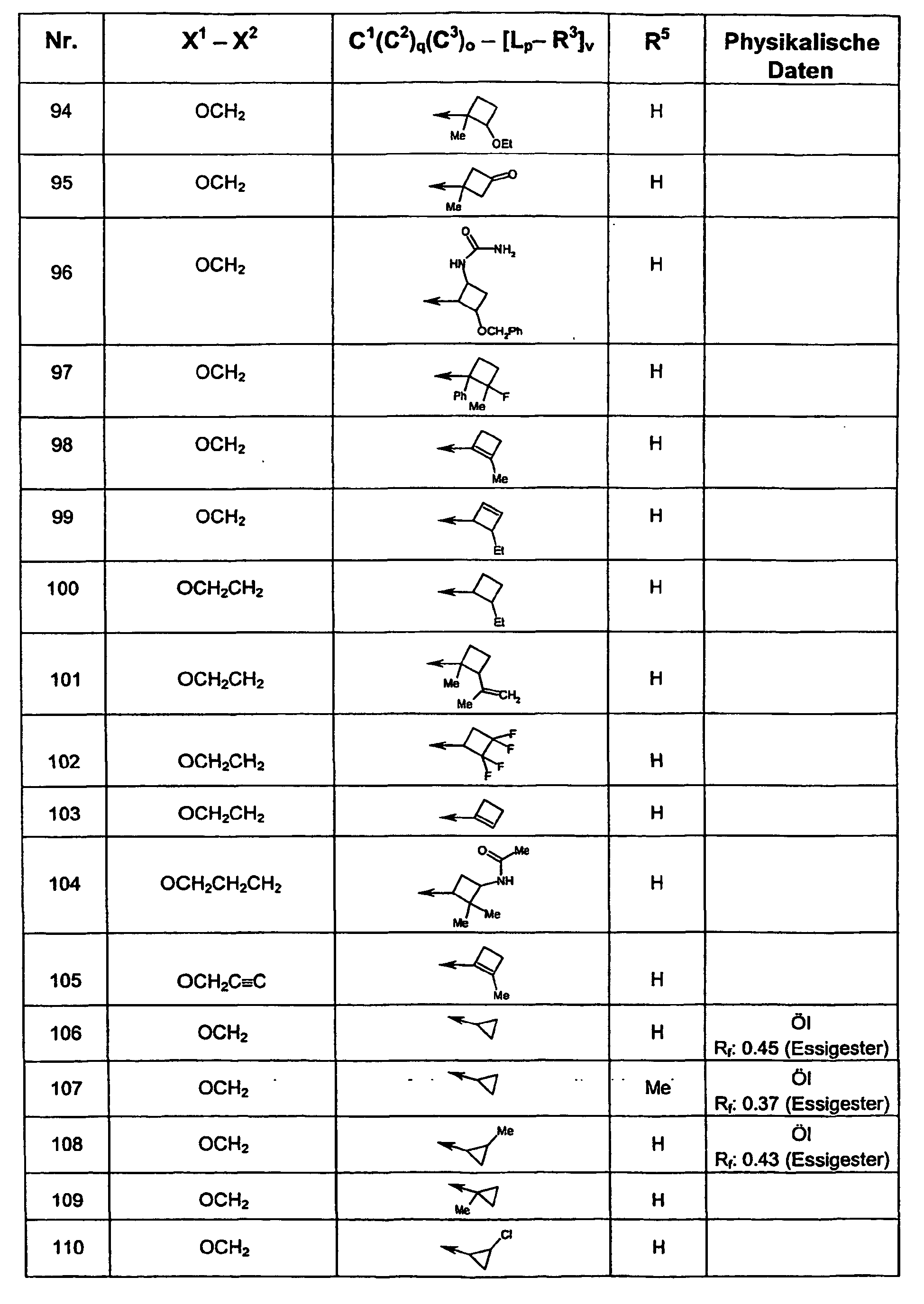

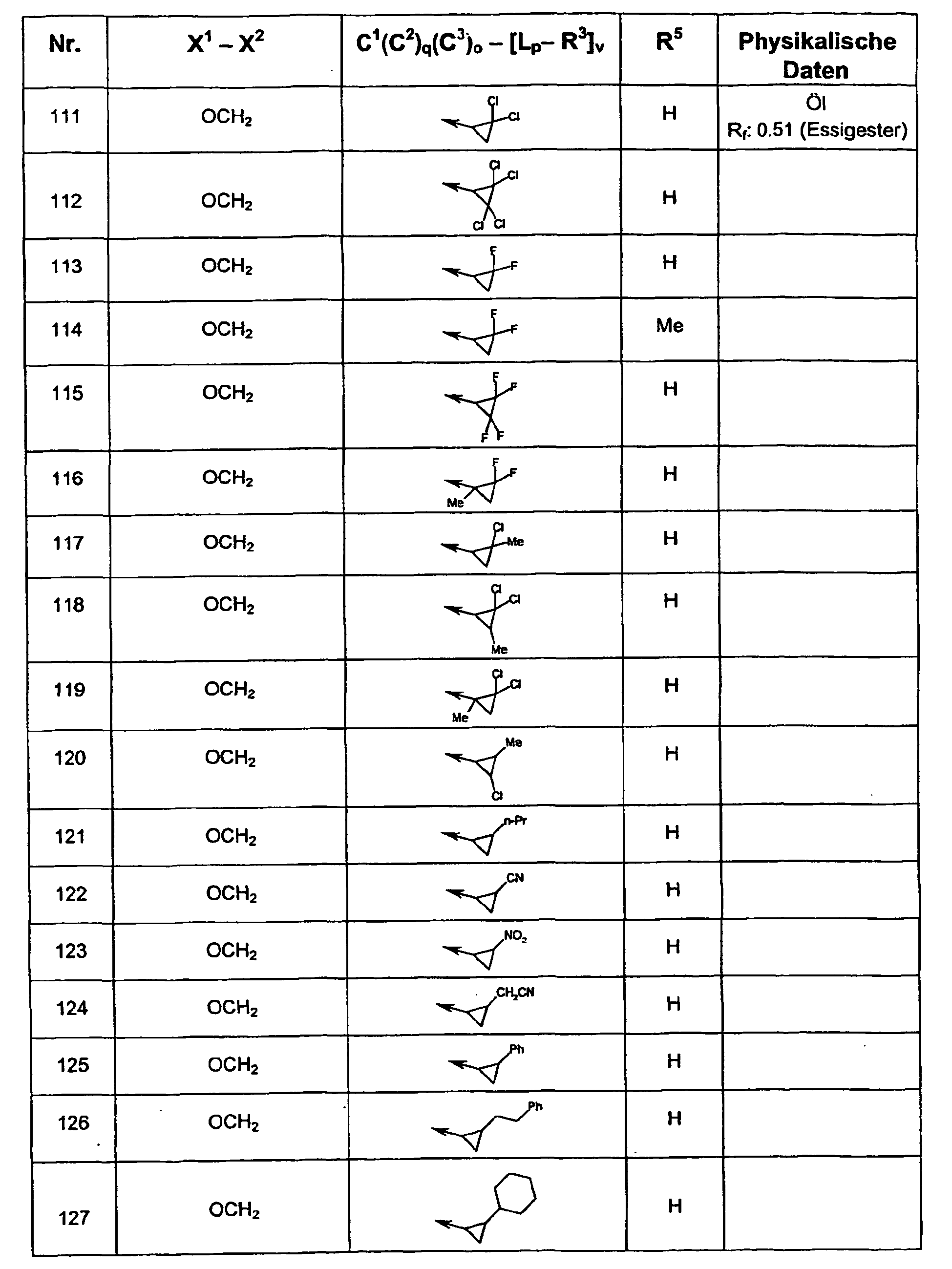

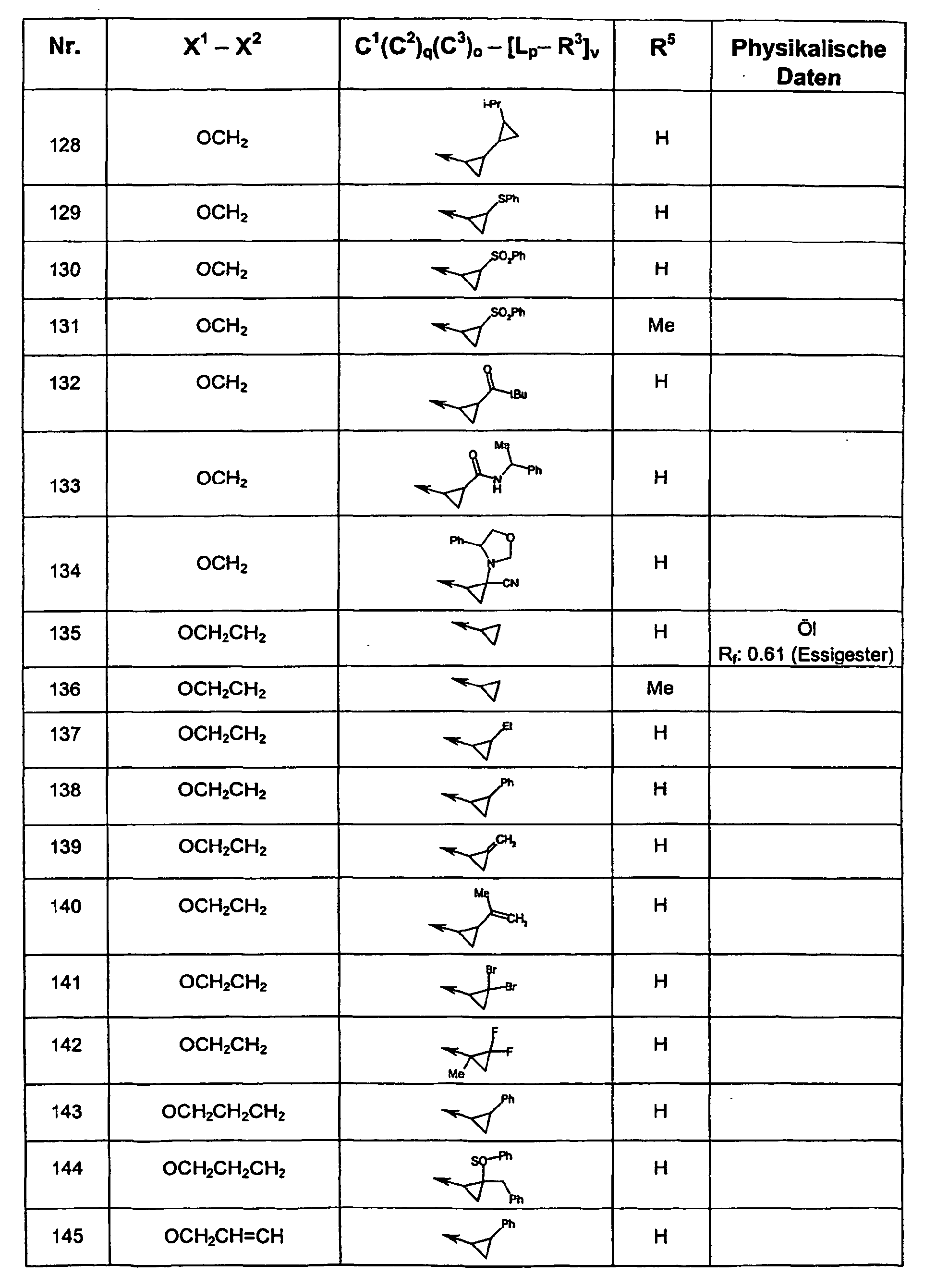

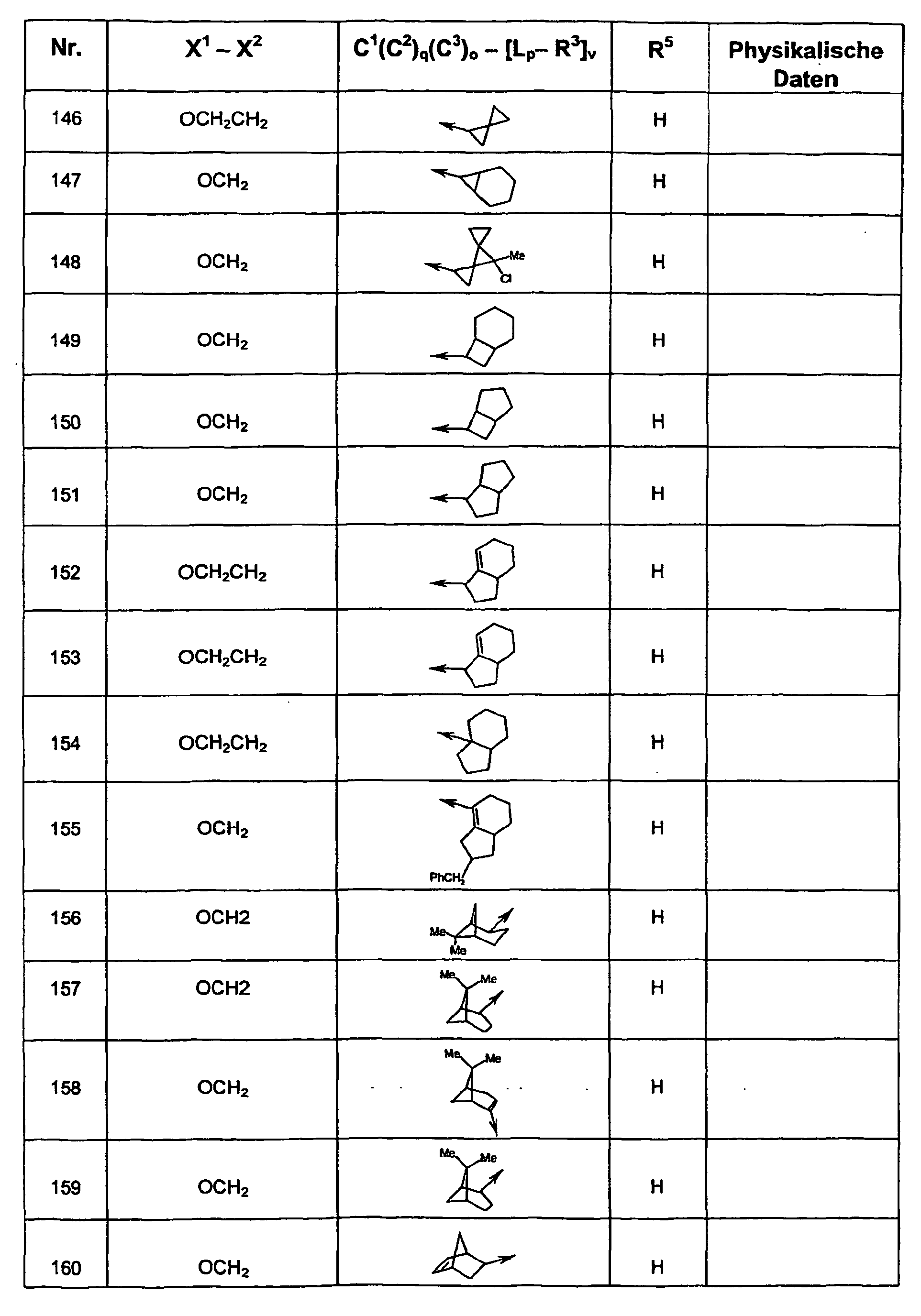

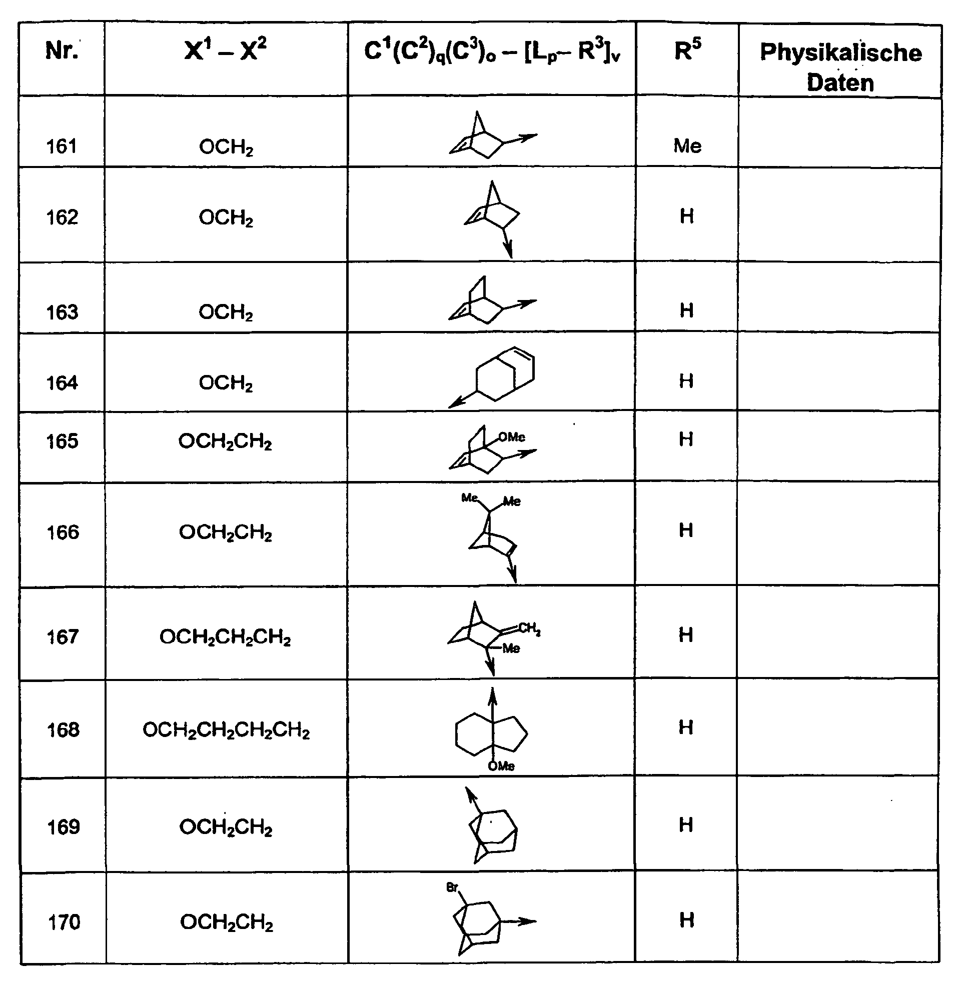

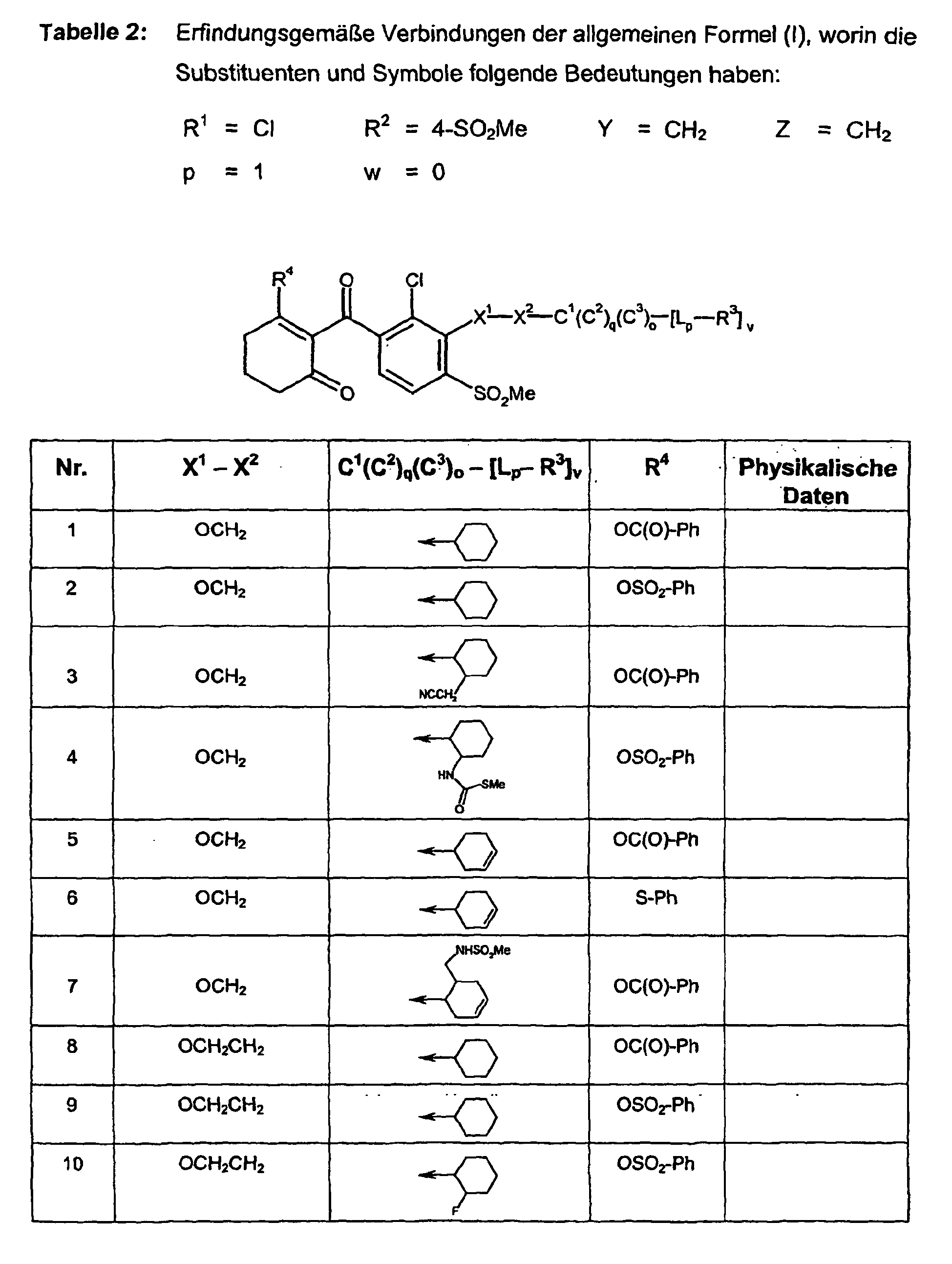

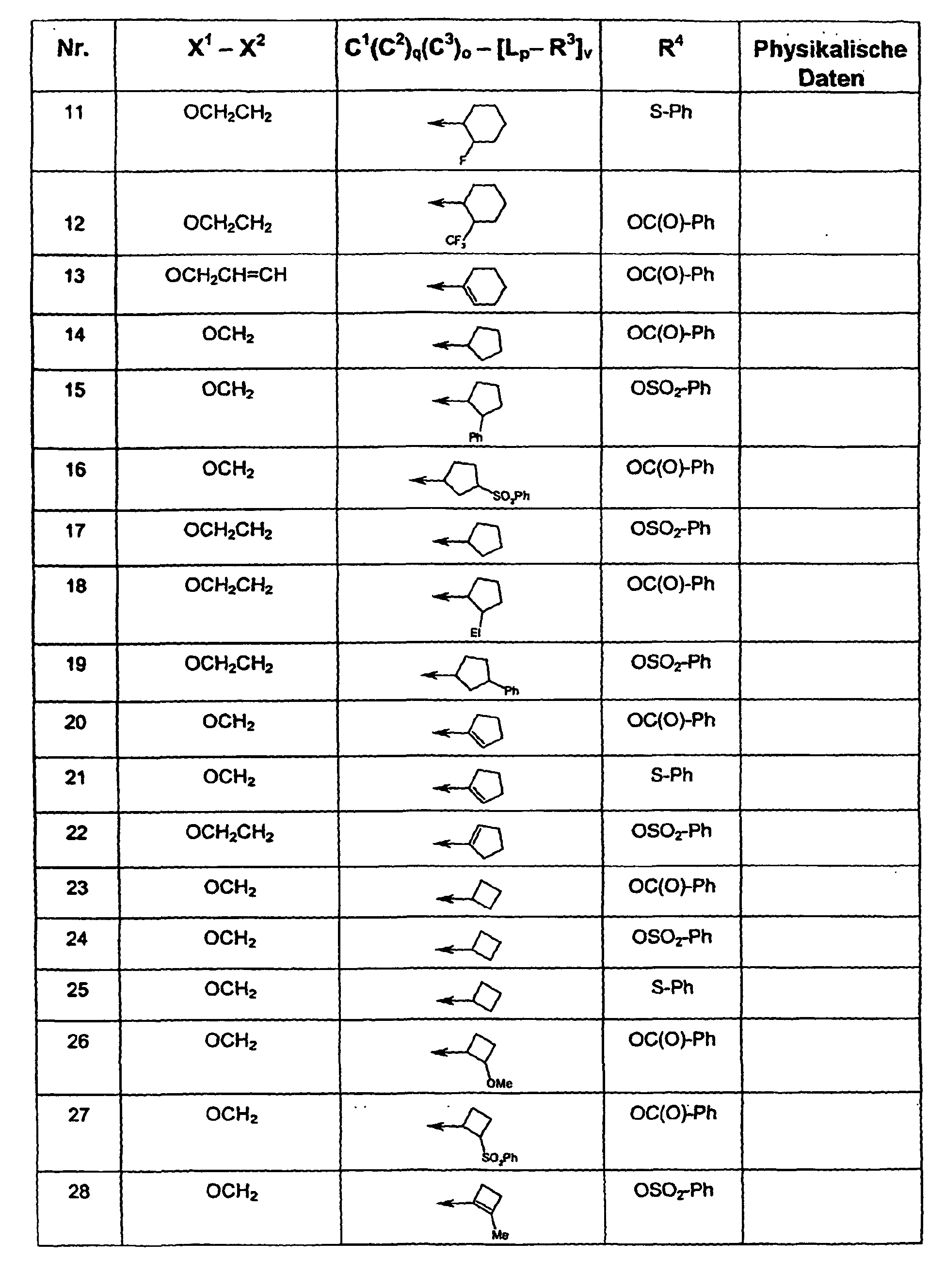

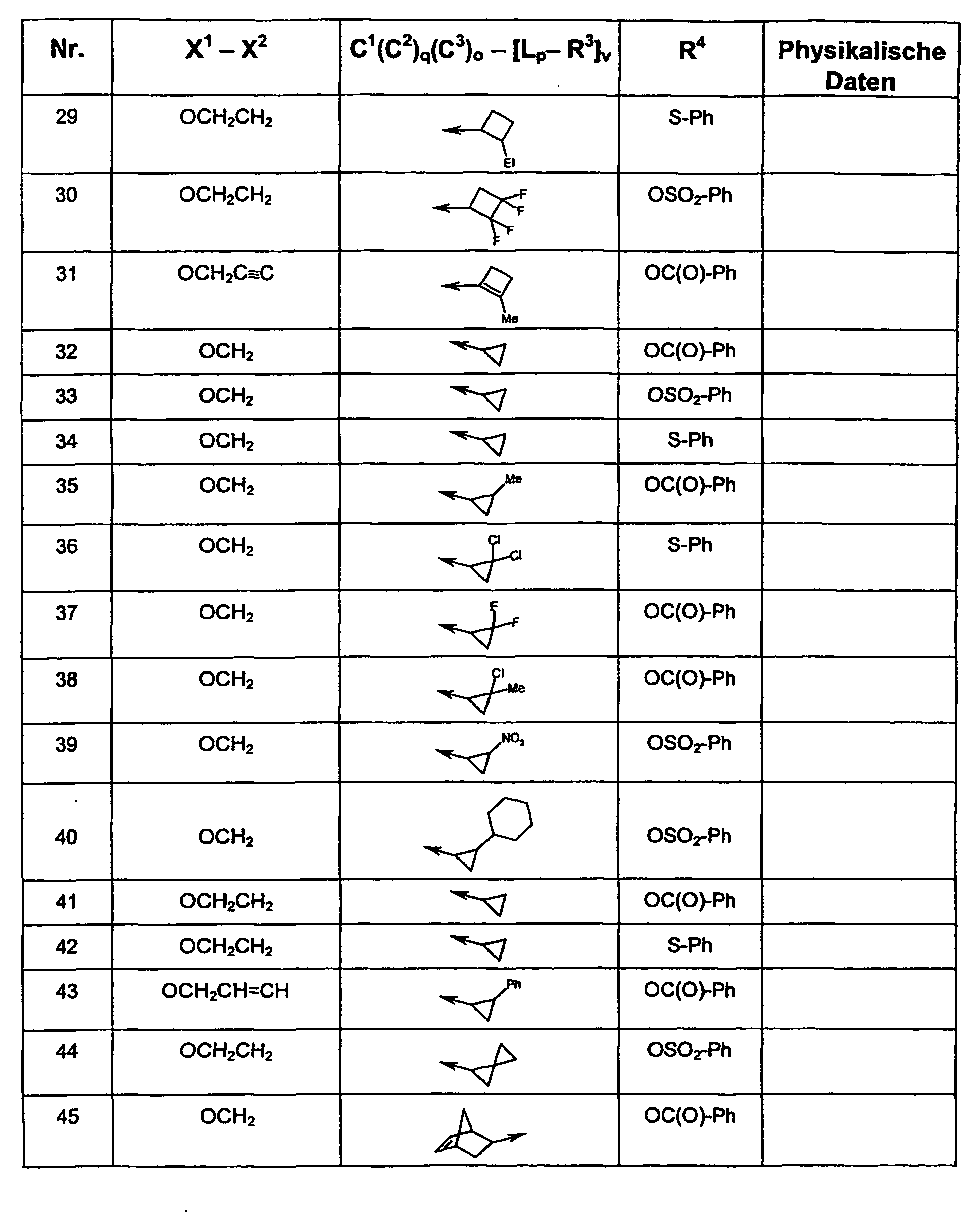

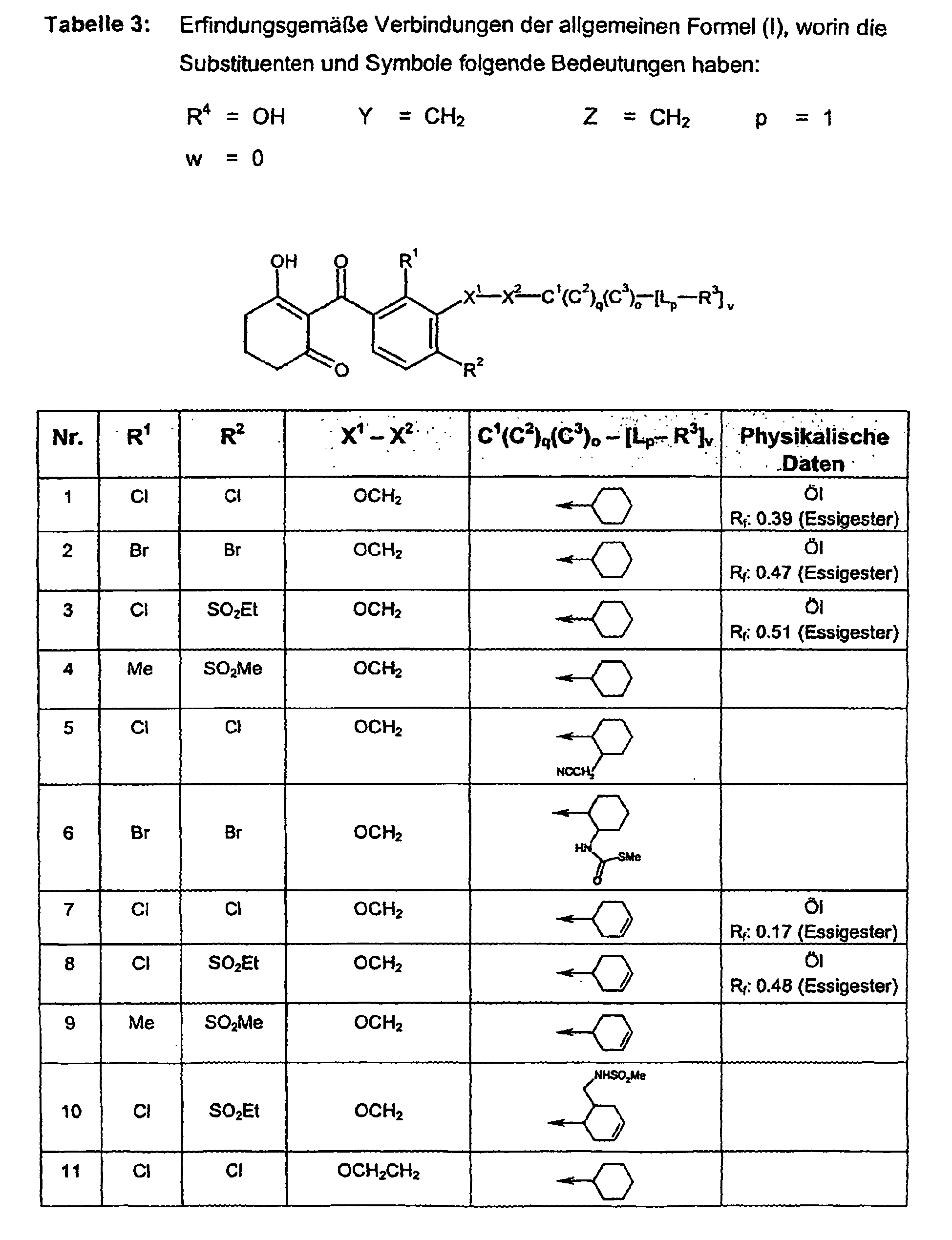

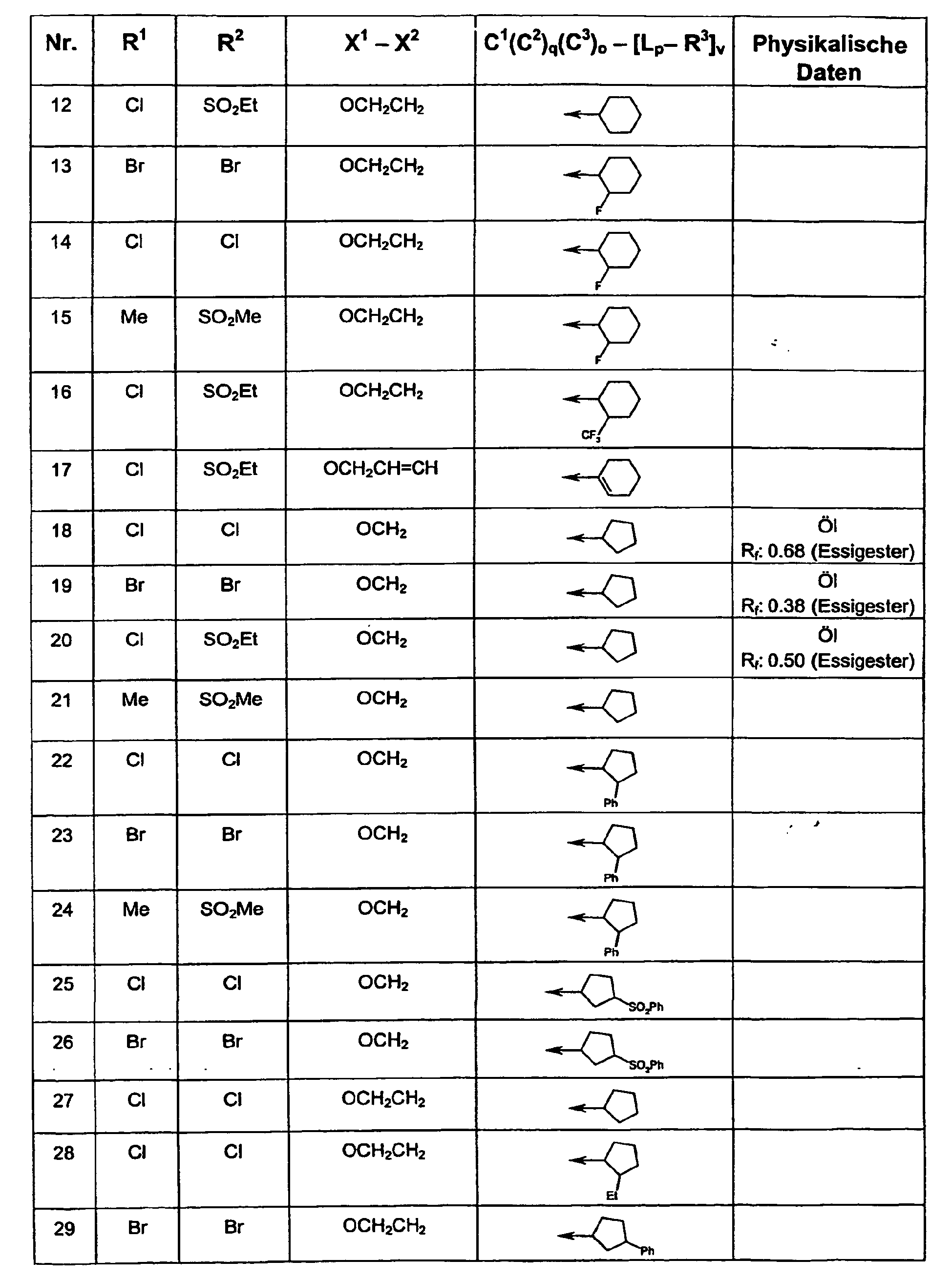

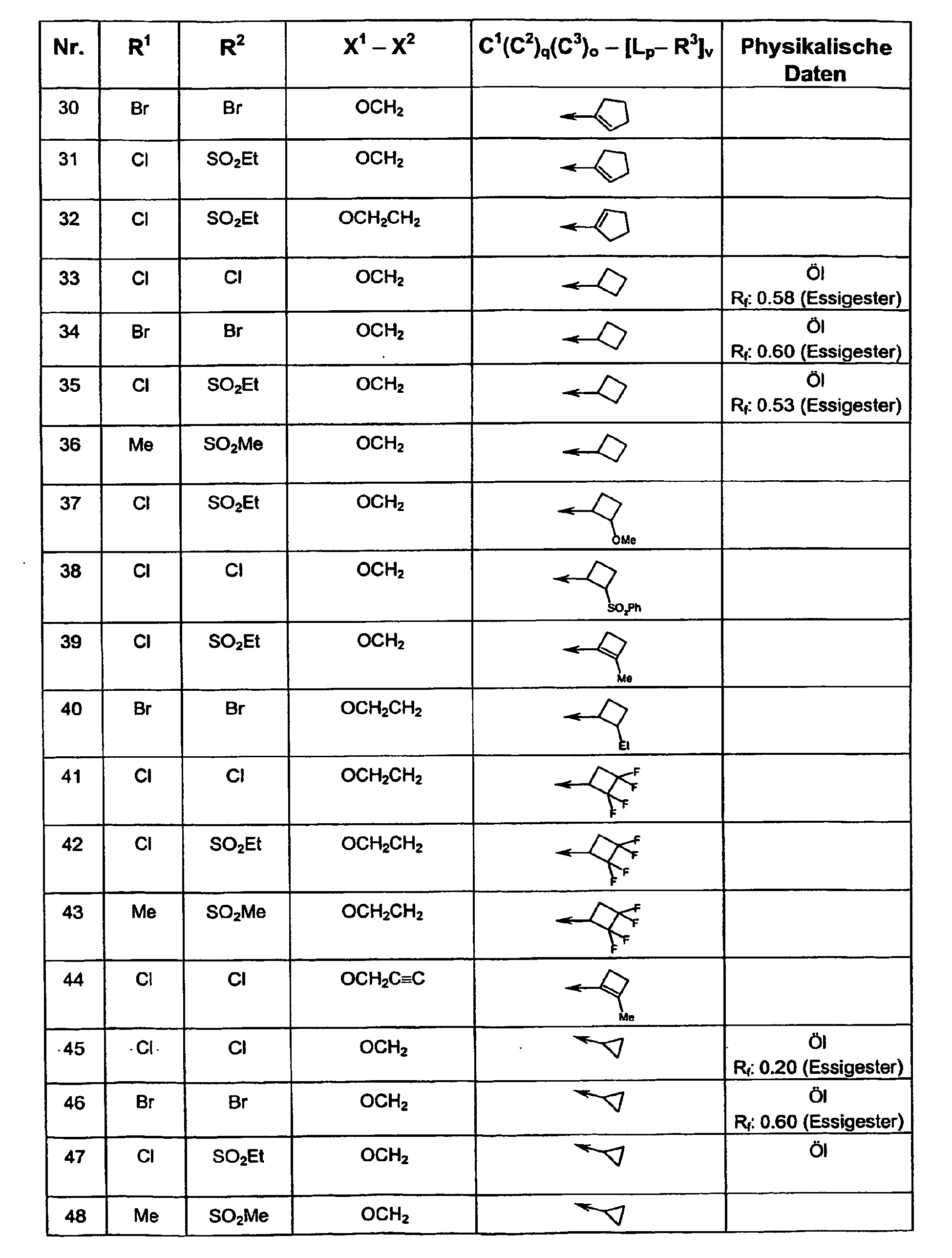

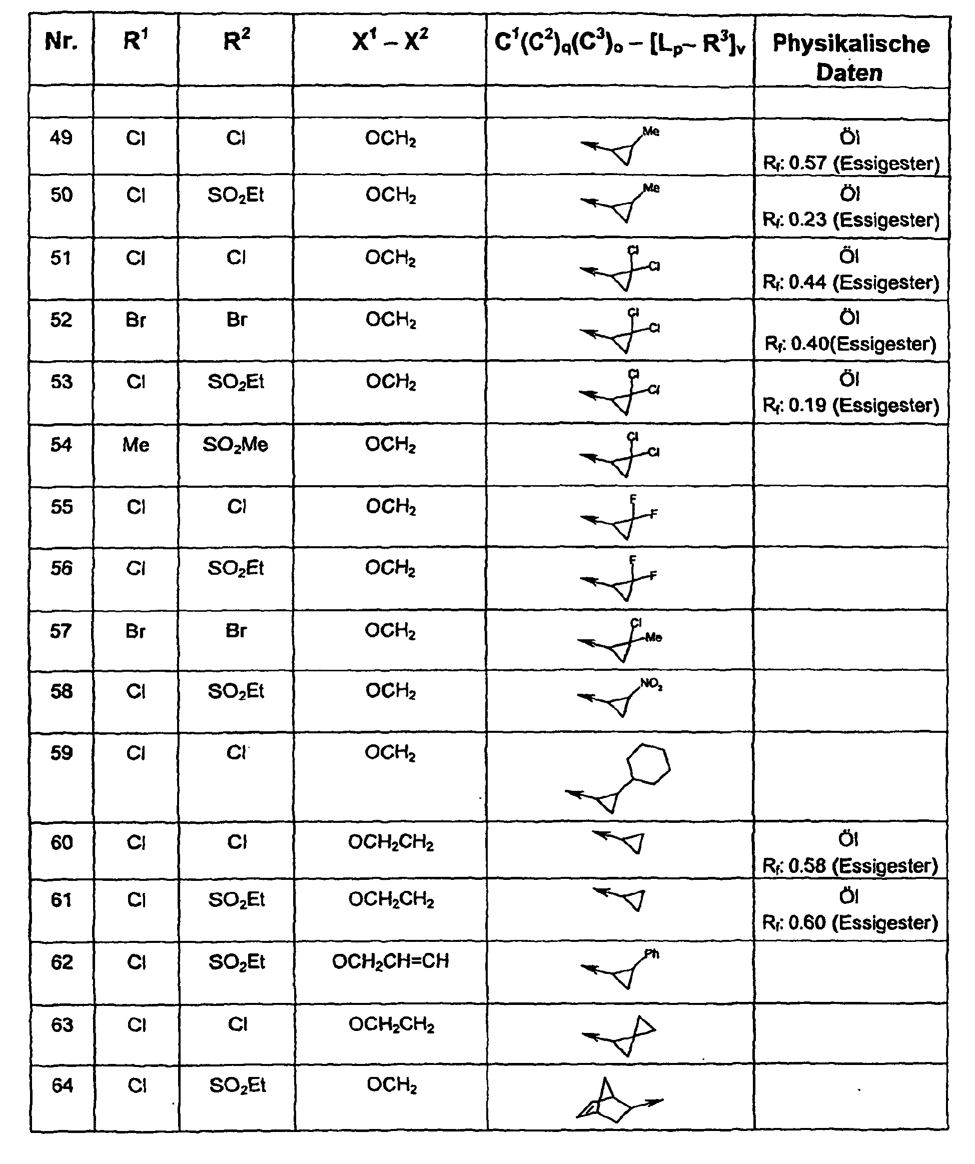

### Formulierungsbeispiele

### 1. Stäubemittel

Ein Stäubemittel wird erhalten, indem man 10 Gew.-Teile einer Verbindung der allgemeinen Formel (I) und 90 Gew.-Teile Talkum als Inertstoff mischt und in einer Schlagmühle zerkleinert.

### 2. Dispergierbares Pulver

Ein in Wasser leicht dispergierbares, benetzbares Pulver wird erhalten, indem man 25 Gewichtsteile einer Verbindung der allgemeinen Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

### 3. Dispersionskonzentrat

Ein in Wasser leicht dispergierbares Dispersionskonzentrat wird erhalten, indem man 20 Gewichtsteile einer Verbindung der allgemeinen Formel (I), 6 Gew.-Teile Alkylphenolpolyglykolether (®Triton X 207), 3 Gew.-Teile Isotridecanolpolyglykolether (8 EO) und 71 Gew.-Teile paraffinischem Mineralöl (Siedebereich z.B. ca. 255 bis über 277°C) mischt und in einer Reibkugelmühle auf eine Feinheit von unter 5 Mikron vermahlt.

### 4. Emulgierbares Konzentrat

Ein emulgierbares Konzentrat wird erhalten aus 15 Gew.-Teilen einer Verbindung der allgemeinen Formel (I), 75 Gew.Teilen Cyclohexanon als Lösemittel und 10 Gew.-Teilen oxethyliertes Nonylphenol als Emulgator.

### 5. Wasserdispergierbares Granulat

Ein in Wasser dispergierbares Granulat wird erhalten, indem man
75 Gew.-Teile einer Verbindung der allgemeinen Formel(I),
10 " ligninsulfonsaures Calcium,
5 " Natriumlaurylsulfat,
3 " Polyvinylalkohol und
7 " Kaolin
mischt, auf einer Stiftmühle mahlt und das Pulver in einem Wirbelbett durch Aufsprühen von Wasser als Granulierflüssigkeit granuliert.

Ein in Wasser dispergierbares Granulat wird auch erhalten, indem man 25 Gew.-Teile einer Verbindung der allgemeinen Formel (I),
5 " 2,2'-dinaphthylmethan-6,6'-disulfonsaures Natrium,
2 " oleoylmethyltaurinsaures Natrium,
1 " Polyvinylalkohol,
17 " Calciumcarbonat und
50 " Wasser
auf einer Kolloidmühle homogenesiert und vorzerkleinert, anschließend auf einer Perimühle mahlt und die so erhaltene Suspension in einem Sprühturm mittels einer Einstoffdüse zerstäubt und trocknet.

### C. Biologische Beispiele

### 1. Herbizide Wirkung im Vorauflauf

Samen von mono- und dikotylen Schadpflanzen werden in Papptöpfen in sandiger Lehmerde ausgelegt und mit Erde abgedeckt. Die in Form von benetzbaren Pulvern oder Emulsionskonzentraten formulierten erfindungsgemäßen Verbindungen werden dann als wäßrige Suspension bzw. Emulsion mit einer Wasseraufwandmenge von umgerechnet 600 bis 800 l/ha in einer Dosierung von umgerechnet 1 kg Aktivsubstanz oder weniger pro Hektar auf die Oberfläche der Abdeckerde appliziert. Nach der Behandlung werden die Töpfe im Gewächshaus aufgestellt und unter guten Wachstumsbedingungen für die Schadpflanzen gehalten. Die optische Bonitur der Pflanzen- bzw. Auflaufschäden erfolgt nach dem Auflaufen der Versuchspflanzen nach einer Versuchszeit von 3 bis 4 Wochen im Vergleich zu unbehandelten Kontrollen. Dabei zeigen beispielsweise die Verbindungen der Beispiele Nr. 2 und 34 aus Tabelle 3 eine mindestens 90%-ige Wirkung gegen Stellaria media und Amaranthus retroflexus.

### 2. Herbizide Wirkung im Nachauflauf

Samen von mono- und dikotylen Schadpflanzen werden in Papptöpfen in sandigem Lehmboden ausgelegt, mit Erde abgedeckt und im Gewächshaus unter guten Wachstumsbedingungen angezogen. Zwei bis drei Wochen nach der Aussaat werden die Versuchspflanzen im Dreiblattstudium behandelt. Die als Spritzpulver bzw. als Emulsionskonzentrate formulierten erfindungsgemäßen Verbindungen werden in einer Dosierung von umgerechnet 1 kg Aktivsubstanz oder weniger pro Hektar mit einer Wasseraufwandmenge von umgerechnet 600 bis 800 I/ha auf die grünen Pflanzenteile gesprüht. Nach 3 bis 4 Wochen Standzeit der Versuchspflanzen im Gewächshaus unter optimalen Wachstumsbedingungen wird die Wirkung der Verbindungen im Vergleich zu unbehandelten Kontrollen bonitiert. Die erfindungsgemäßen Verbindungen weisen auch im Nachauflauf eine sehr gute herbizide Wirksamkeit gegen ein breites Spektrum wirtschaftlich wichtiger mono- und dikotyler Schadpflanzen auf. Dabei zeigen beispielsweise die Verbindungen der Beispiele Nr. 2 und 34 aus Tabelle 3 eine mindestens 90%-ige Wirkung gegen Stellaria media.

### 3. Wirkung auf Schadpflanzen in Reis

Typische Schadpflanzen in Reiskulturen werden im Gewächshaus unter Paddyreis-Bedingungen (Anstauhöhe des Wassers: 2-3 cm) angezogen. Nach der Behandlung mit den formulierten erfindungsgemäßen Verbindungen in einer Dosierung von umgerechnet 1 kg Aktivsubstanz oder weniger pro Hektar werden die Versuchspflanzen im Gewächshaus unter optimalen Wachstumsbedingungen aufgestellt und während der gesamten Versuchszeit so gehalten. Etwa drei Wochen nach der Applikation erfolgt die Auswertung mittels optischer Bonitur der Pflanzenschäden im Vergleich zu unbehandelten Kontrollen. Die erfindungsgemäßen Verbindungen weisen sehr gute herbizide Wirkung gegen Schadpflanzen auf. Dabei zeigen beispielsweise die Verbindungen der Beispiele Nr. 34 und 46 aus Tabelle 3 eine mindestens 90%-ige Wirkung gegen Cyperus difformis und Echinochloa crus galli.

### 4. Kulturpflanzenverträglichkeit

In weiteren Versuchen im Gewächshaus werden Samen einer größeren Anzahl von Kulturpflanzen und mono- und dikotyler Schadpflanzen in sandigem Lehmboden ausgelegt und mit Erde abgedeckt. Ein Teil der Töpfe wird sofort wie unter Punkt 1 beschrieben behandelt, die übrigen im Gewächshaus aufgestellt, bis die Pflanzen zwei bis drei echte Blätter entwickelt haben und dann wie unter Punkt 2 beschrieben mit den erfindungsgemäßen Verbindungen der Formel (I) in unterschiedlichen Dosierungen besprüht. Vier bis fünf Wochen nach der Applikation und Standzeit im Gewächshaus wird mittels optischer Bonitur festgestellt, daß die erfindungsgemäßen Verbindungen dikotyle Kulturen wie z.B. Soja und Zuckerrüben im Vor- und Nachauflaufverfahren in der Regel selbst bei hohen Wirkstoffdosierungen ungeschädigt oder nahezu ungeschädigt lassen. Einige Substanzen schonen darüber hinaus auch Gramineen-Kulturen wie z.B. Gerste, Weizen und Reis. Die Verbindungen der Formel (I) zeigen teilsweise eine hohe Selektivität und eignen sich deshalb zur Bekämpfung von teilweise unerwünschtem Pflanzenwuchs in landwirtschaftlichen Kulturen.

## Patentansprüche

1. Verbindungen der Formel (I) oder deren Salze 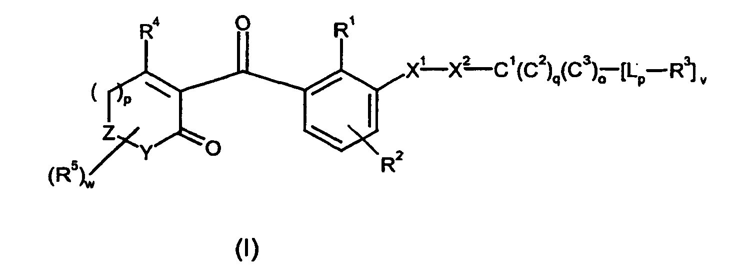 worin
X¹ eine divalente Einheit aus der Gruppe O, S(O)ₙ, NH, N[Lₚ- R³];
X² eine geradkettige oder verzweigte, durch w Halogenatome und durch k Reste [Lₚ-R³] substituierte (C₁-C₆)-Alkylen-, (C₂-C₆)-Alkenylen- oder (C₂-C₆)-Alkinylenkette;
C¹(C²)_{q}(C³)ₒ einen mono-, bi- oder tricyclischer Rest, wobei
c) die Ringe C¹, C² und C³ jeweils für einen 3- bis 8-gliedrigen, gesättigten oder teilgesättigten Ring aus der Gruppe Cycloalkyl, Cycloalkenyl, Oxiranyl und Oxetanyl stehen,
a) die Ringe C¹, C² und C³ jeweils untereinander über ein oder zwei gemeinsame Atome verknüpft sind;
R¹ und R² unabhängig voneinander Wasserstoff, Mercapto, Nitro, Cyano, Halogen, Thiocyanato, (C₁-C₆)-Alkyl-CO-O, (C₁-C₆)-Alkyl-S(O)ₙ-O, (C₁-C₆)-Alkyl-S(O)ₙ, Di-(C₁-C₆)-Alkyl-NH-SO₂, (C₁-C₆)-Alkyl-SO₂-NH, (C₁-C₆)-Alkyl-NH-CO, (C₁-C₆)-Alkyl-SO₂[(C₁-C₆)-Alkyl]amino, (C₁-C₆)-Alkyl-CO-(C₁-C₆)-Alkyl)-amino, 1,2,4,-Triazol-1-yl, (C₁-C₆)-Alkyl-O-CH₂, (C₁-C₆)-Alkyl-S(O)ₙ-CH₂. (C₁-C₆)-Alkyl-NH-CH₂, [(C₁-C₆)-Alkyl]₂N-CH₂, 1,2,4,-Triazol-1-yl-CH₂, durch v Reste aus der Gruppe Cyano, Nitro und Halogen substituiertes (C₁-C₆)-Alkyl-(D)ₚ, (C₂-C₆)-Alkenyl-(D)p, (C₂-C₆)-Alkinyl-(D)p, (C₃-C₉)-Cycloalkyl-(D)p, (C₃-C₉)-Cycloalkenyl-(D)ₚ, (C₁-C₆)-Alkyl-(C₃-C₉)-Cycloalkyl-(D)ₚ oder(C₁-C₆)-Alkyl-(C₃-C₉)-Cycloalkenyl-(D)ₚ;
R³ Wasserstoff, Hydroxy, Halogen, Mercapto, Amino, Nitro, ein kohlenstoffhaltiger Rest oder, falls p in X¹ für null steht, Oxo, NR⁸, N-OR⁸ oder N-NR⁸R⁹;
D Sauerstoff oder Schwefel;
L jeweils geradkettiges oder verzweigtes Aₚ-[C(R⁶)₂]_{w}-[Aₚ-C(R⁶)₂]ₓ-Aₚ oder Ap-M-Ap;
mit der Maßgabe, daß 2 oder 3 der Laufzahlen p, w und x nicht gleichzeitig null sein sollen;
A eine divalente Einheit aus der Gruppe O S(O)ₙ, NH, N-(C₁-C₆)-Alkyl, N-(C₂-C₆)-Alkenyl und N-(C₂-C₆)-Alkinyl;
M durch w Reste R⁶ substituiertes (C₁-C₆)-Alkylen, (C₂-C₆)-Alkenylen oder (C₂-C₆)-Alkinylen;
R⁴ OR⁷, (C₁-C₄)-Alkylthio, Halogen-(C₁-C₄)-alkylthio, (C₁-C₄)-Alkenylthio, Halogen-(C₂-C₄)-alkenylthio, (C₂-C₄)-Alkinylthio, Halogen-(C₂-C₄)-alkinylthio, (C₂-C₄)-Alkylsulfinyl, Halogen-(C₂-C₄)-alkylsulfinyl, (C₂-C₄)-Alkenylsulfinyl, Halogen-(C₂-C₄)-alkenylsulfinyl, (C₂-C₄)-Alkinylsulfinyl, Halogen-(C₂-C₄)-alkinylsulfinyl, (C₁-C₄)-Alkylsulfonyl, Halogen-(C₁-C₄)-alkylsulfonyl, (C₂-C₄)-Alkenylsulfonyl, Halogen-(C₂-C₄)-alkenylsulfonyl, (C₂-C₄)-Alkinylsulfonyl, Halogen-(C₂-C₄)-alkinylsulfonyl, Cyano, Cyanato, Thiocyanato, Halogen oder Phenylthio;
R⁵ Wasserstoff, Tetrahydropyranyl-3, Tetrahydropyranyl-4, Tetrahydrothiopyranyl-3, (C₁-C₄)-Alkyl, (C₃-C₈)-Cycloalkyl, (C₁-C₄)-Alkoxy, (C₁-C₄)-Alkoxy-(C₁-C₄)--alkyl, (C₁-C₄)-Alkylcarbonyl, (C₁-C₄)-Alkoxycarbonyl, (C₁-C₄)-Alkylthio, Phenyl, wobei die acht letztgenannten Gruppen durch v Reste aus der Gruppe Halogen, (C₁-C₄)-Alkylthio und (C₁-C₄)-Alkoxy substituiert sind, oder zwei an einem gemeinsamen Kohlenstoffatom gebundene Reste R⁵ bilden eine Kette aus der Gruppe OCH₂CH₂O, OCH₂CH₂CH₂O, SCH₂CH₂S und SCH₂CH₂CH₂S, wobei diese durch w Methylgruppen substituiert ist, oder zwei an direkt benachbarten Kohlenstoffatomen gebundene Reste R⁵ bilden mit den sie tragenden Kohlenstoffatomen einen durch w Reste aus der Gruppe Halogen, (C₁-C₄)-Alkyl, (C₁-C₄)-Alkylthio und (C₁-C₄)-Alkoxy substituierten 3- bis 6-gliedrigen Ring;
R⁶ (C₁-C₄)-Alkyl, Halogen, Cyano oder Nitro;
R⁷ Wasserstoff, (C₁-C₄)-Alkyl, Halogen-(C₁-C₄)-alkyl, (C₁-C₄)-Alkoxy-(C₁-C₄)-alkyl, Formyl, (C₁-C₄)-Alkylcarbonyl, (C₁-C₄)-Alkoxycarbonyl, (C₁-C₄)-Alkylaminocarbonyl, Di-(C₁-C₄)-alkylaminocarbonyl, (C₁-C₄)-Alkylsulfonyl, Halogen-(C₁-C₄)-alkylsulfonyl, Benzoyl oder Phenylsulfonyl, wobei die beiden letztgenannten Gruppen durch v Reste aus der Gruppe (C₁-C₄)-Alkyl, Halogen-(C₁-C₄)-alkyl, (C₁-C₄)-Alkoxy, Halogen-(C₁-C₄)-alkoxy, Halogen, Cyano und Nitro substituiert sind;
R⁸ Wasserstoff, (C₁-C₄)-Alkyl, (C₁-C₄)-Alkoxy, (C₂-C₄)-Alkenyl, (C₂-C₄)-Alkinyl, (C₃-C₈)-Cycloalkyl, Aryl, Aryl-(C₁-C₆)-alkyl, Heteroaryl, Heterocyclyl, Halogen-(C₁-C₄)-alkyl;
R⁹ Wasserstoff, (C₁-C₄)-Alkyl, (C₂-C₄)-Alkenyl, (C₂-C₄)-Alkinyl, (C₃-C₉)-Cycloalkyl, Aryl, Aryl-(C₁-C₆)-alkyl, Heteroaryl, Heterocyclyl, Halogen-(C₁-C₄)-alkyl, oder sofern R⁸ und R⁹ an einem Atom oder an zwei direkt benachbarten Atomen gebunden sind, bilden sie gemeinsam mit den sie bindenden Atomen einen gesättigten, teilweise oder vollständig ungesättigten fünf- bis sechsgliedrigen Ring, der p Heteroatome aus der Gruppe Sauerstoff, Stickstoff und Schwefel enthält;
Y eine divalente Einheit aus der Gruppe O, S, N-H, N-(C₁-C₄)-Alkyl, CHR⁵ und C(R⁵)₂;
Z eine divalente Einheit aus der Gruppe O, S, SO, SO₂, N-H, N-(C₁-C₄)-Alkyl, CHR⁵ und C(R⁵)₂;
m und n jeweils 0, 1 oder 2;
o, p und q jeweils 0 oder 1;
w und x jeweils 0,1, 2, 3 oder 4;
v 0, 1, 2 oder 3 bedeuten.

2. Benzoylcyclohexandione nach Anspruch 1, worin
X¹ eine divalente Einheit aus der Gruppe O, S und NH;
R¹ Chlor, Brom, Fluor, Methyl, Ethyl, Cyano, Nitro, Halogen-(C₁-C₂)-alkyl;
R² Halogen, Halogen-(C₁-C₄)-alkyl, (C₁-C₄)-Alkylsulfenyl, (C₁-C₄)-Alkylsulfinyl, (C₁-C₄)-Alkylsulfonyl oder Nitro;
R⁵ (C₁-C₄)-Alkyl, (C₃-C₈)-Cycloalkyl, (C₁-C₄)-Alkoxy, (C₁-C₄)-Alkoxy-(C₁-C₄)-alkyl, (C₁-C₄)-Alkylcarbonyl, (C₁-C₄)-Alkoxycarbonyl, (C₁-C₄)-Alkylthio, Phenyl, oder zwei an einem gemeinsamen Kohlenstoffatom gebundene Reste R⁵ bilden eine Kette aus der Gruppe OCH₂CH₂O, OCH₂CH₂CH₂O, SCH₂CH₂S und SCH₂CH₂CH₂S, wobei diese durch w Methylgruppen substituiert ist, oder zwei an direkt benachbarten Kohlenstoffatomen gebundene Reste R⁵ bilden eine Bindung oder bilden mit den sie tragenden Kohlenstoffatomen einen durch w Reste aus der Gruppe Halogen, (C₁-C₄)-Alkyl, (C₁-C₄)-Alkylthio und (C₁-C₄)-Alkoxy substituierten 3- bis 6-gliedrigen Ring;
R⁸ Wasserstoff, (C₁-C₄)-Alkyl, (C₁-C₄)-Alkoxy, (C₂-C₄)-Alkenyl, (C₂-C₄)-Alkinyl, (C₃-C₈)-Cycloalkyl, Aryl, Aryl-(C₁-C₆)-alkyl, Halogen-(C₁-C₄)-alkyl;
R⁹ Wasserstoff, (C₁-C₄)-Alkyl, oder sofern R⁸ und R⁹ an einem Atom oder an zwei direkt benachbarten Atomen gebunden sind, bilden sie gemeinsam mit den sie bindenden Atomen einen gesättigten, teilweise oder vollständig ungesättigten fünf-bis sechsgliedrigen Ring, der p Heteroatome aus der Gruppe Sauerstoff, Stickstoff und Schwefel enthält.

3. Benzoylcyclohexandione nach Anspruch 1 oder 2, worin
X² eine geradkettige oder verzweigte, durch w Halogenatome substituierte (C₁-C₄)-Alkylen-, (C₂-C₄)-Alkenylen- oder (C₂-C₄)-Alkinylenkette;
R³
a) Wasserstoff, Hydroxy, Halogen, Mercapto, Amino, Nitro, Cyano, Formyl,
b) durch w Reste aus der Gruppe Halogen, Cyano, (C₁-C₄)-Alkyl, Halogen-(C₁-C₄)-alkyl, (C₁-C₄)-Alkoxy, Halogen-(C₁-C₄)-alkoxy, (C₁-C₄)-Alkylthio, Halogen-(C₁-C₄)-alkylthio und R¹⁰ substituiertes Phenyl, Oxazolyl, Furanyl oderTetrahydropyrrolyl,
c) durch v Reste aus der Gruppe Formyl, Halogen, Cyano, Nitro, (C₁-C₄)-Alkylamino, (C₁-C₄)-Dialkylamino, (C₁-C₄)-Alkoxycarbonyl, (C₁-C₄)-Alkylcarbonyl, (C₁-C₄)-Alkylcarbonyloxy, (C₁-C₄)-Alkyl, (C₂-C₄)-Alkenyl, (C₂-C₄)-Alkinyl, Halogen-(C₁-C₄)-alkyl, (C₁-C₄)-Alkylthio, Halogen-(C₁-C₄)-alkylthio, (C₁-C₄)-Alkoxy und Halogen-(C₁-C₄)-alkoxy substituiertes (R¹¹)(C₁-C₄)-Alkylamino, (R¹¹)₂-Amino, R¹¹-Oxycarbonyl, R¹¹-Carbonyl, R¹¹-Carbonyloxy; (C₁-C₆)-Alkyl, (C₂-C₆)-Alkenyl, (C₂-C₆)-Alkinyl, (C₁-C₆)-Alkoxy-(C₁-C₆)-alkyl, (C₂-C₆)-Alkinyloxy-(C₁-C₆)-alkyl, (C₃-C₉)-Cycloalkyl, (C₃-C₉)-Cyloalkenyl, (C₁-C₆)-Alkoxy oder (C₁-C₆)-Alkylthio;
d) ein Rest der Formel Va, Vb, Vc, Vd, Vj oder Vp, oder
e) falls p für null steht, Oxo, NR⁸, N-OR⁸ oder N-NR⁸R⁹;
R⁷ Wasserstoff, (C₁-C₄)-Alkylsulfonyl, Benzoyl oder Phenylsulfonyl, wobei die beiden letztgenannten Gruppen durch v Reste aus der Gruppe (C₁-C₂)-Alkyl, Halogen-(C₁-C₂)-alkyl, (C₁-C₂)-Alkoxy, Halogen-(C₁-C₂)-alkoxy, Halogen, Cyano und Nitro substituiert sind, und
R¹¹ Wasserstoff, (C₁-C₄)-Alkyl, (C₂-C₄)-Alkenyl, (C₂-C₄)-Alkinyl oder (C₃-C₈)-Cycoalkyl bedeuten.

4. Benzoylcyclohexandione nach einem der Ansprüche 1 bis 3, worin
X¹ die divalente Einheit O;
R⁴ OR⁷, (C₁-C₄)-Alkylthio, (C₂-C₄)-Alkenylthio, (C₁-C₄)-Alkylsulfonyl, Cyano, Cyanato, Thiocyanato oder durch v Reste aus der Gruppe Halogen, (C₁-C₂)-Alkyl, (C₁-C₂)-Alkoxy, Halogen-(C₁-C₂)-alkyl, Halogen-(C₁-C₂)-alkoxy und Nitro substituiertes Phenylthio;
R⁵ Wasserstoff, (C₁-C₄)-Alkyl, (C₃-C₈)-Cycloalkyl, (C₁-C₄)-Alkoxy, (C₁-C₄)-Alkylthio, Phenyl, oder zwei an direkt benachbarten Kohlenstoffatomen gebundene Reste R⁵ bilden mit den sie tragenden Kohlenstoffatomen einen substituierten 3- bis 6-gliedrigen Ring;
R¹² Wasserstoff, (C₁-C₄)-Alkyl, (C₂-C₄)-Alkenyl, oder sofern R¹¹ und R¹² an einem Atom oder an zwei direkt benachbarten Atomen gebunden sind, bilden sie gemeinsam mit den sie bindenden Atomen einen gesättigten, teilweise oder vollständig ungesättigten fünf- bis sechsgliedrigen Ring, der p Heteroatome aus der Gruppe Sauerstoff, Stickstoff und Schwefel enthält;
Y eine divalente Einheit aus der Gruppe CHR⁵ und C(R⁵)₂, und
Z eine divalente Einheit aus der Gruppe O, S, SO₂, N-(C₁-C₄)-Alkyl, CHR⁵ und C(R⁵)₂ bedeuten.

5. Benzoylcyclohexandione nach einem der Ansprüche 1 bis 4, worin
R² Halogen, Halogen-(C₁-C₂)-alkyl oder (C₁-C₂)-Alkylsulfonyl;
R⁵ (C₁-C₄)-Alkyl, (C₃-C₈)-Cycloalkyl, (C₁-C₄)-Alkoxy, (C₁-C₄)-Alkylthio, Phenyl, oder zwei an direkt benachbarten Kohlenstoffatomen gebundene Reste R⁵ bilden mit den sie tragenden Kohlenstoffatomen einen substituierten 3- bis 6-gliedrigen Ring;
R⁷ Wasserstoff, (C₁-C₄)-Alkylsulfonyl, Benzoyl oder Phenylsulfonyl, und
R⁸ Wasserstoff, Methyl oder Ethyl bedeuten, und
R² in 4-Position des Phenylrings steht.

6. Benzoylcyclohexandione nach einem der Ansprüche 1 bis 5, worin
X² eine geradkettige oder verzweigte (C₁-C₄)-Alkylen-, (C₂-C₄)-Alkenylen- oder (C₂-C₄)-Alkinylenkette;
R¹ Chlor, Brom, Methyl, Trifluormethyl, Cyano oder Nitro;
R² Chlor, Brom, Methylsulfonyl, Ethylsulfonyl, Trifluormethyl oder Nitro;
R⁴ OR⁷, (C₁-C₄)-Alkylthio, (C₂-C₄)-Alkenylthio oder Phenylthio;
R⁵ Wasserstoff, (C₁-C₄)-Alkyl, oder zwei an direkt benachbarten Kohlenstoffatomen gebundene Reste R⁵ bilden mit den sie tragenden Kohlenstoffatomen einen substituierten 3- bis 6-gliedrigen Ring;
A eine divalente Einheit aus der Gruppe O, S(O)ₙ, NH und N-(C₁-C₆)-Alkyl;
M (C₁-C₆)-Alkylen;
Y und Z unabhängig voneinander eine divalente Einheit aus der Gruppe, CHR⁵ und C(R⁵)₂ bedeuten.

7. Herbizide Mittel, **gekennzeichnet durch** einen herbizid wirksamen Gehalt an mindestens einer Verbindung der allgemeinen Formel (I) gemäß einem der Ansprüche 1 bis 6.

8. Herbizide Mittel nach Anspruch 7 in Mischung mit Formulierungshilfsmittein.

9. Verfahren zur Bekämpfung unerwünschter Pflanzen, **dadurch gekennzeichnet, daß** man eine wirksame Menge mindestens einer Verbindung der allgemeinen Formel (I) nach einem der Ansprüche 1 bis 6 oder eines herbiziden Mittels nach Anspruch 7 oder 8 auf die Pflanzen oder auf den Ort des unerwünschten Pflanzenwachstums appliziert.

10. Verwendung von Verbindungen der allgemeinen Formel (I) nach einem der Ansprüche 1 bis 6 oder von herbiziden Mitteln nach Anspruch 7 oder 8 zur Bekämpfung unerwünschter Pflanzen.

11. Verwendung nach Anspruch 10, **dadurch gekennzeichnet, daß** die Verbindungen der allgemeinen Formel (I) zur Bekämpfung unerwünschter Pflanzen in Kulturen von Nutzpflanzen eingesetzt werden.

12. Verwendung nach Anspruch 11, **dadurch gekennzeichnet, daß** die Nutzpflanzen transgene Nutzpflanzen sind.

## Claims

1. A compound of the formula (I) or a salt thereof 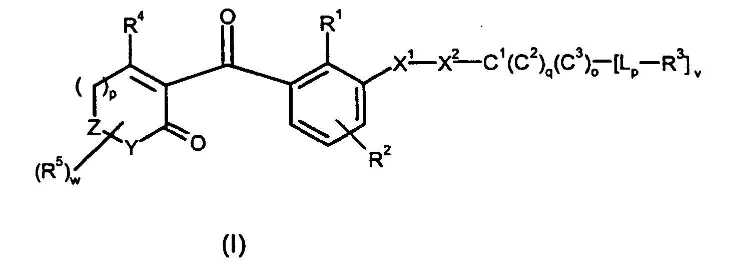 in which
X¹ is a divalent unit selected from the group consisting of O, S(O)ₙ, NH, N[Lₚ― R³];
X² is a straight-chain or branched (C₁-C₆)-alkylene, (C₂-C₆)-alkenylene or (C₂-C₆)-alkynylene chain which is substituted by w halogen atoms and by k radicals [Lₚ-R³];
C¹(C²)_{q}(C³)ₒ is a mono-, bi- or tricyclic radical, where
a) the rings C¹, C² and C³ are in each case a 3- to 8-membered, saturated or partially saturated ring selected from the group consisting of cycloalkyl, cycloalkenyl, oxiranyl and oxetanyl,
b) the rings C¹, C² and C³ are in each case linked to each other via one or two joint atoms;
R¹ and R² independently of one another are hydrogen, mercapto, nitro, cyano, halogen, thiocyanato, (C₁-C₆)-alkyl-CO-O, (C₁-C₆)-alkyl-S(O)ₙ-O, (C₁-C₆)-alkyl-S(O)ₙ, di-(C₁-C₆)-alkyl-NH-SO₂, (C₁-C₆)-alkyl-SO₂-NH, (C₁₋C₆)-alkyl-NH-CO, (C₁-C₆)-alkyl-SO₂-[(C₁-C₆)-alkyl]amino, (C₁-C₆)-alkyl-CO-((C₁-C₆)-alkyl)amino, 1,2,4-triazol-1-yl, (C₁-C₆)-alkyl-O-CH₂, (C₁-C₆)-alkyl-S(O)ₙ-CH₂, (C₁-C₆)-alkyl-NH-CH₂, [(C₁-C₆)-alkyl]₂N-CH₂, 1,2,4-triazol-1-yl-CH₂, or are (C₁-C₆)-alkyl-(D)p, (C₂-C₆)-alkenyl-(D)p. (C₂-C₆)-alkynyl-(D)p, (C₃-C₉)-cycloalkyl-(D)ₚ, (C₃-C₉)-cycloalkenyl-(D)ₚ, (C₁-C₆)-alkyl-(C₃-C₉)-cycloalkyl-(D)p or (C₁-C₆)-alkyl-(C₃-C₉)-cycloalkenyl-(D)ₚ, each of which is substituted by v radicals selected from the group consisting of cyano, nitro and halogen;
R³ is hydrogen, hydroxyl, halogen, mercapto, amino, nitro, a carbon-containing radical or, if p in X¹ is zero, R³ is oxo, NR⁸, N-OR⁸ or N-NR⁸R⁹;
D is oxygen or sulfur;
L is in each case straight-chain or branched Aₚ-[C(R⁶)₂]_{w}-[Aₚ-C(R⁶)₂]ₓ₋Ap or
Ap-M-Ap;
with the proviso that 2 or 3 of the variable terms p, w and x shall not simultaneously be zero;
A is a divalent unit selected from the group consisting of O, S(O)ₙ, NH, N-(C₁-C₆)-alkyl, N-(C₂-C₆)-alkenyl and N-(C₂-C₆)-alkynyl;
M is (C₁-C₆)-alkylene, (C₂-C₆)-alkenylene or (C₂-C₆)-alkynylene, each of which is substituted by w radicals R⁶;
R⁴ is OR⁷, (C₁-C₄)-alkylthio, halo-(C₁-C₄)-alkylthio, (C₁-C₄)-alkenylthio, halo-(C₂-C₄)-alkenylthio, (C₂-C₄)-alkynylthio, halo-(C₂-C₄)-alkynylthio, (C₂₋C₄)-alkylsulfinyl, halo-(C₂-C₄)-alkylsulfinyl, (C₂-C₄)-alkenylsulfinyl, halo-(C₂₋C₄)-alkenylsulfinyl, (C₂-C₄)-alkynylsulfinyl, halo-(C₂-C₄)-alkynylsulfinyl, (C₁--C₄)-alkylsulfonyl, halo-(C₁-C₄)-alkylsulfonyl, (C₂-C₄)-alkenylsulfonyl, halo-(C₂-C₄)-alkenylsulfonyl, (C₂-C₄)-alkynylsulfonyl, halo-(C₂-C₄)-alkynylsulfonyl, cyano, cyanato, thiocyanato, halogen or phenylthio;
R⁵ is hydrogen, tetrahydropyran-3-yl, tetrahydropyran-4-yl, tetrahydrothio-pyran-3-yl, (C₁-C₄)-alkyl, (C₃-C₈)-cycloalkyl, (C₁-C₄)-alkoxy, (C₁-C₄)-alkoxy-(C₁-C₄)-alkyl, (C₁-C₄)-alkylcarbonyl, (C₁-C₄)-alkoxycarbonyl, (C₁-C₄)-alkylthio, phenyl, the eight last-mentioned groups being substituted by v radicals selected from the group consisting of halogen, (C₁-C₄)-alkylthio and (C₁-C₄)-alkoxy, or
two radicals R⁵ bonded to a joint carbon atom form a chain selected from the group consisting of OCH₂CH₂O, OCH₂CH₂CH₂O, SCH₂CH₂S and SCH₂CH₂CH₂S, this group being substituted by w methyl groups, or
two radicals R⁵ bonded to directly adjacent carbon atoms, together with the carbon atoms to which they are attached, form a 3- to 6-membered ring which is substituted by w radicals selected from the group consisting of halogen, (C₁-C₄)-alkyl, (C₁-C₄)-alkylthio and (C₁-C₄)-alkoxy;
R⁶ is (C₁-C₄)-alkyl, halogen, cyano or nitro;
R⁷ is hydrogen, (C₁-C₄)-alkyl, halo-(C₁-C₄)-alkyl, (C₁-C₄)-alkoxy-(C₁-C₄)-alkyl, formyl, (C₁-C₄)-alkylcarbonyl, (C₁-C₄)-alkoxycarbonyl, (C₁-C₄)-alkylaminocarbonyl, di-(C₁-C₄)-alkylaminocarbonyl, (C₁-C₄)-alkylsulfonyl, halo-(C₁-C₄)-alkylsulfonyl benzoyl or phenylsulfonyl, the two last-mentioned groups being substituted by v radicals selected from the group consisting of (C₁-C₄)-alkyl, halo-(C₁-C₄)-alkyl, (C₁-C₄)-alkoxy, halo-(C₁-C₄)-alkoxy, halogen, cyano and nitro;
R⁸ is hydrogen, (C₁-C₄)-alkyl, (C₁-C₄)-alkoxy, (C₂-C₄)-alkenyl, (C₂-C₄)-alkynyl, (C₃-C₈)-cycloalkyl, aryl, aryl-(C₁-C₆)-alkyl, heteroaryl, heterocyclyl, halo-(C₁-C₄)-alkyl;
R⁹ is hydrogen, (C₁-C₄)-alkyl, (C₂-C₄)-alkenyl, (C₂-C₄)-alkynyl, (C₃-C₉)-cycloalkyl, aryl, aryl-(C₁-C₆)-alkyl, heteroaryl, heterocyclyl, halo-(C₁-C₄)--alkyl, or, if R⁸ and R⁹ are bonded to one atom or to two directly adjacent atoms, they together with the atoms to which they are bonded form a saturated, partially or fully unsaturated five- to six-membered ring which contains p hetero atoms selected from the group consisting of oxygen, nitrogen and sulfur;
Y is a divalent unit selected from the group consisting of O, S, N-H, N-(C₁-C₄)-alkyl, CHR⁵ and C(R⁵)₂;
Z is a divalent unit selected from the group consisting of O, S, SO, SO₂, N-H, N-(C₁-C₄)-alkyl, CHR⁵ and C(R⁵)₂;
m and n are each 0, 1 or 2;
o, p and q are each 0 or 1;
w and x are each 0, 1, 2, 3 or 4;
v is 0, 1, 2 or 3.

2. A benzoylcyclohexanedione as claimed in claim 1, in which
X¹ is a divalent unit selected from the group consisting of O, S and NH;
R¹ is chlorine, bromine, fluorine, methyl, ethyl, cyano, nitro, halo-(C₁₋C₂)-alkyl;
R² is halogen, halo-(C₁-C₄)-alkyl, (C₁-C₄)-alkylsulfenyl, (C₁-C₄)-alkylsulfinyl, (C₁-C₄)-alkylsulfonyl or nitro;
R⁵ is (C₁-C₄)-alkyl, (C₃-C₈)-cycloalkyl, (C₁-C₄)-alkoxy, (C₁-C₄)-alkoxy-(C₁-C₄)-alkyl, (C₁-C₄)-alkylcarbonyl, (C₁-C₄)-alkoxycarbonyl, (C₁-C₄)-alkylthio, phenyl, or
two radicals R⁵ bonded to a joint carbon atom form a chain selected from the group consisting of OCH₂CH₂O, OCH₂CH₂CH₂O, SCH₂CH₂S and SCH₂CH₂CH₂S, this group being substituted by w methyl groups, or two radicals R⁵ bonded to directly adjacent carbon atoms form a bond or, together with the carbon atoms to which they are attached, form a 3- to 6-membered ring which is substituted by w radicals selected from the group consisting of halogen, (C₁-C₄)-alkyl, (C₁-C₄)-alkylthio and (C₁-C₄)-alkoxy;
R⁸ is hydrogen, (C₁-C₄)-alkyl, (C₁-C₄)-alkoxy, (C₂-C₄)-alkenyl, (C₂-C₄)-alkynyl, (C₃-C₈)-cycloalkyl, aryl, aryl-(C₁-C₆)-alkyl, halo-(C₁-C₄)-alkyl;
R⁹ is hydrogen, (C₁-C₄)-alkyl, or, if R⁸ and R⁹ are bonded to one atom or to two directly adjacent atoms, they together with the atoms to which they are bonded form a saturated, partially or fully unsaturated five- to six-membered ring which contains p hetero atoms selected from the group consisting of oxygen, nitrogen and sulfur.

3. A benzoylcyclohexanedione as claimed in claim 1 or 2, in which
X² is a straight-chain or branched (C₁-C₄)-alkylene, (C₂-C₄)-alkenylene or (C₂-C₄)-alkynylene chain, each of which is substituted by w halogen atoms;
R³ is
a) hydrogen, hydroxyl, halogen, mercapto, amino, nitro, cyano, formyl,
b) phenyl, oxazolyl, furanyl or tetrahydropyrrolyl, each of which is substituted by w radicals selected from the group consisting of halogen, cyano, (C₁-C₄)-alkyl, halo-(C₁-C₄)-alkyl, (C₁-C₄)-alkoxy, halo-(C₁-C₄)-alkoxy, (C₁-C₄)-alkylthio, halo-(C₁-C₄)-alkylthio and R¹⁰,
c) (R¹¹)(C₁-C₄)-alkylamino, (R¹¹)₂-amino, R¹¹-oxycarbonyl, R¹¹⁻carbonyl, R¹¹-carbonyloxy; (C₁-C₆)-alkyl, (C₂-C₆)-alkenyl, (C₂-C₆)-alkynyl, (C₁-C₆)-alkoxy-(C₁-C₆)-alkyl, (C₂-C₆)-alkynyloxy-(C₁-C₆)-alkyl, (C₃-C₉)-cycloalkyl, (C₃-C₉)-cyloalkenyl, (C₁-C₆)-alkoxy or (C₁₋C₆)-alkylthio, each of which is substituted by v radicals selected from the group consisting of formyl, halogen, cyano, nitro, (C₁-C₄)--alkylamino, (C₁-C₄)-dialkylamino, (C₁-C₄)-alkoxycarbonyl, (C₁-C₄)-alkylcarbonyl, (C₁-C₄)-alkylcarbonyloxy, (C₁-C₄)-alkyl, (C₂-C₄)-alkenyl, (C₂-C₄)-alkynyl, halo-(C₁-C₄)-alkyl, (C₁-C₄)-alkylthio, halo-(C₁-C₄)-alkylthio, (C₁-C₄)-alkoxy and halo-(C₁-C₄)-alkoxy;
d) a radical of the formula Va, Vb, Vc, Vd, Vj or Vp, or
e) if p is zero, oxo, NR⁸, N-OR⁸ or N-NR⁸R⁹;
R⁷ is hydrogen, (C₁-C₄)-alkylsulfonyl, benzoyl or phenylsulfonyl, the two last-mentioned groups being substituted by v radicals selected from the group consisting of (C₁-C₂)-alkyl, halo-(C₁-C₂)-alkyl, (C₁-C₂)-alkoxy, halo-(C₁-C₂)-alkoxy, halogen, cyano and nitro, and
R¹¹ is hydrogen, (C₁-C₄)-alkyl, (C₂-C₄)-alkenyl, (C₂-C₄)-alkynyl or (C₃₋C₈)-cycloalkyl.

4. A benzoylcyclohexanedione as claimed in any of claims 1 to 3, in which
X¹ is the divalent unit O;
R⁴ is OR⁷, (C₁-C₄)-alkylthio, (C₂-C₄)-alkenylthio, (C₁-C₄)-alkylsulfonyl, cyano, cyanato, thiocyanato, or else phenylthio which is substituted by v radicals selected from the group consisting of halogen, (C₁-C₂)-alkyl, (C₁₋C₂)-alkoxy, halo-(C₁-C₂)-alkyl, halo-(C₁-C₂)-alkoxy and nitro;
R⁵ is hydrogen, (C₁-C₄)-alkyl, (C₃-C₈)-cycloalkyl, (C₁-C₄)-alkoxy, (C₁₋C₄)-alkylthio, phenyl, or two radicals R⁵ bonded to directly adjacent carbon atoms, together with the carbon atoms to which they are bonded, form a substituted 3- to 6-membered ring;
R¹² is hydrogen, (C₁-C₄)-alkyl, (C₂-C₄)-alkenyl, or, if R¹¹ and R¹² are bonded to one atom or to two directly adjacent atoms, they together with the atoms to which they are bonded form a saturated, partially or fully unsaturated five- to six-membered ring which contains p hetero atoms selected from the group consisting of oxygen, nitrogen and sulfur;
Y is a divalent unit selected from the group consisting of CHR⁵ and C(R⁵)₂, and
Z is a divalent unit selected from the group consisting of O, S, SO₂, N-(C₁-C₄)-alkyl, CHR⁵ and C(R⁵)₂.

5. A benzoylcyclohexanedione as claimed in any of claims 1 to 4, in which
R² is halogen, halo-(C₁-C₂)-alkyl or (C₁-C₂)-alkylsulfonyl;
R⁵ is (C₁-C₄)-alkyl, (C₃-C₈)-cycloalkyl, (C₁-C₄)-alkoxy, (C₁-C₄)-alkylthio, phenyl, or two radicals R⁵ bonded to directly adjacent carbon atoms together with the carbon atoms to which they are attached form a substituted 3- to 6-membered ring;
R⁷ is hydrogen, (C₁-C₄)-alkysulfonyl, benzoyl or phenylsulfonyl, and
R⁸ is hydrogen, methyl or ethyl, and
R² is in the 4-position of the phenyl ring.

6. 4. A benzoylcyclohexanedione as claimed in any of claims 1 to 35, in which
X² is a straight-chain or branched (C₁-C₄)-alkylene, (C₂-C₄)-alkenylene or (C₂-C₄)-alkynylene chain;
R¹ is chlorine, bromine, methyl, trifluoromethyl, cyano or nitro;
R² is chlorine, bromine, methylsulfonyl, ethylsulfonyl, trifluoromethyl or nitro;
R⁴ is OR⁷, (C₁-C₄)-alkylthio, (C₂-C₄)-alkenylthio or phenylthio;
R⁵ is hydrogen, (C₁-C₄)-alkyl, or two radicals R⁵ bonded to directly adjacent carbon atoms together with the carbon atoms to which they are attached form a substituted 3- to 6-membered ring;
A is a divalent unit selected from the group consisting of O, S(O)ₙ, NH and N-(C₁-C₆)-alkyl;
M is (C₁-C₆)-alkylene;
Y and Z independently of one another are a divalent unit selected from the group consisting of CHR⁵ and C(R⁵)₂.

7. A herbicidal composition which comprises a herbicidally active content of at least one compound of the formula (I) as claimed in any of claims 1 to 65.

8. A herbicidal composition as claimed in claim 76 in mixture with formulation auxiliaries.

9. A method of controlling undesired plants, which comprises applying an effective amount of at least one compound of the formula (I) as claimed in any of claims 1 to 5 6 or of a herbicidal composition as claimed in claim 67 or 8 to the plants or to the site of the undesired plant growth.

10. The use of compounds of the formula (I) as claimed in any of claims 1 to 46 or of herbicidal compositions as claimed in claim 76 or 78 for controlling undesired plants.

11. The use as claimed in claim 910, wherein the compounds of the formula (I) are employed for controlling undesired plants in crops of useful plants.

12. The use as claimed in claim 11, wherein the useful plants are transgenic useful plants.

## Revendications

1. Composés de formule (1) ou leurs sels 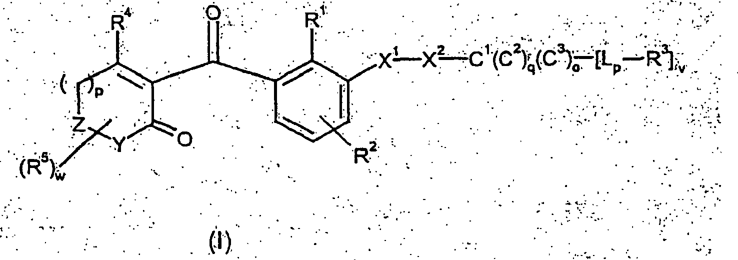 dans laquelle
X¹ représente une unité bivalente prise parmi les groupes O, S(O)ₙ, NH, N[Lp―R³];
X² représente une chaîne alkylène en C₁-C₆, alcénylène en, C₂-C₆ ou alcynylène en C₂-C₆ linéaire ou ramifiée, substituée par w atomes d'halogène ou par k restes [Lp―R³] ;
C¹(C²)_{q}(C³)ₒ représente un reste mono-, bi- ou tricyclique,
c) les cycles C¹, C² et C³ à chaque fois représentant un cycle à 3 à 8 chaînons, saturé ou partiellement saturé, pris parmi les groupe cycloalkyle, cycloalcényle, oxiranyle et oxétanyle,
a) les cycles C¹, C² et C³ étant à chaque fois reliés entre eux par un ou deux atomes communs ;
R¹ et R² indépendamment l'un de l'autre, représentent un atome d'hydrogène, un groupe mercapto, nitro, cyano, halogène, thiocyanato, (alkyl en C₁-C₆)-CO-O-, (alkyl en C₁-C₆) -S (O)ₙ-O, (alkyl en C₁-C₆) -S(O)ₙ, di- ((alkyl en C₁-C₆)-NH-SO₂, (alkyl en C₁-C₆)-SO₂-NH, (alkyl en C₁-C₆)-NH-CO, (alkyl en C₁-C₆) -SO₂-(alkyl en C₁-C₆) -amino, (alkyl en C₁-C₆)-CO-(alkyl en C₁-C₆)-amino, 1,2,4-triazol-1-yle, (alkyl en C₁-C₆)-O-CH₂, (alkyl en C₁-C₆)- S(O)ₙ-CH₂, (alkyl en C₁-C₆)-NH-CH₂, (alkyl en C₁-C₆)₂-N-CH₂, 1,2,4-triazol-1-yl-CH₂, (alkyl en C₁-C₆)-(D)ₚ, (alcényl en C₂-C₆)-(D)ₚ, (alcynyl en C₂-C₆)-(D)ₚ, cyclo(alkyl en C₃-C₉)-(D)ₚ, cyclo(alcényl en C₃-C₉)- (D)ₚ, (alkyl en C₁-C₆)-cyclo (alkyl en C₃-C₉)-(D)ₚ ou (alkyl en C₁-C₆)-cyclo (alcényl C₃-C₉)-(D)ₚ substitué par v restes pris dans le groupe des cyano, nitro et halogène ;
R³ représente un atome d'hydrogène, un groupe hydroxy, halogène, mercapto, amino, nitro, un reste hydrocarboné ou, dans le cas où p dans X¹ vaut zéro, un groupe oxo, NR⁸, N-OR⁸ ou N-NR⁸R⁹ ;
D représente un atome d'oxygène ou de soufre ;
L à chaque fois représente un groupe Ap-[C(R⁶)₂]_{w}-[Aₚ-C(R⁶)₂]ₓ-Aₚ ou Ap-M-Ap linéaire ou ramifié ;
à condition que 2 ou 3 des nombres courants p, w et x ne soient pas simultanément égaux à zéro ;
A représente une unité bivalente prise parmi les groupes O, S(O)ₙ, NH, N-(alkyle en C₁-C₆), N-(alcényle en C₂-C₆) et N-(alcynyle en C₂-C₆) ;
M représente un groupe alkylène en C₁-C₆, alcénylène en C₂-C₆ ou alcynylène en C₂-C₆ substitué par w restes R⁶ ;
R⁴ représente un groupe OR⁷, (alkyl en C₁-C₄)thio, halogèno-(alkyl en C₁-C₄) thio, (alcényl en C₁-C₄) -thio, halogéno-(alcényl en C₂-C₄) thio, (alcynyl en C₂-C₄) thio, halogéno (alcynyl en C₂-C₄)-thio, (alkyl en C₂-C₄) sulfinyle, halogéno (alkyl en C₂-C₄) sulfinyle, (alcényl en C₂-C₄)sulfinyle, halogéno(alcényl en C₂-C₄)-sulfinyle, (alcynyl en C₂-C₄) sulfinyle, halogéno (alcynyl en C₂-C₄)sulfinyle, (alkyl en C₁-C₄)sulfonyle, halogéno(alkyl en C₁-C₄)-sulfonyle, (alcényl en C₂-C₄) sulfonyle, halogéno (alcényl en C₂-C₄) sulfonyle, (alcynyl en C₂-C₄)sulfonyle, halogéno(alcynyl en C₂-C₄)-sulfonyle, cyano, cyanato, thiocyanato, halogène ou phénylthio ;
R⁵ représente un atome d'hydrogène, un groupe tétrahydropyranyle-3, tétrahydropyranyle-4, tétrahydrothiopyranyle-3, alkyle en C₁-C₄, cycloalkyle en C₃-C₈, alkoxy en C₁-C₄, (alkoxy en C₁-C₄) -alkyle en C₁-C₄, (alkyl en C₁-C₄) carbonyle, (alkoxy en C₁-C4) carbonyle, (alkyl en C₁-C₄)thio, phényle, les huit derniers groupes cités étant substitués par v restes pris parmi les groupes halogène, (alkyl en C₁-C₄) thio et alkoxy en C₁₋C₄, ou
deux restes R⁵ liés à un atome de carbone commun forment une chaîne prise parmi les groupes OCH₂CH₂O, OCH₂CH₂CH₂O, SCH₂CH₂S et SCH₂CH₂CH₂S, celle-ci étant substituée par w groupes méthyle, ou
deux restes R⁵ liés à des atomes de carbone directement voisins forment avec les atomes de carbone qui les portent un cycle à 3 à 6 chaînons substitué par w restes pris parmi les groupes halogène, alkyle en C₁-C₄, (alkyl en C₁-C₄)-thio et alkoxy en C₁-C₄ ;
R⁶ représente un groupe alkyle en C₁-C₄, halogène, cyano ou nitro ;
R⁷ représente un atome d'hydrogène, un groupe alkyle en C₁-C₄, halogénoalkyle en C₁-C₄, (alkoxy en C₁-C₄) -alkyle en C₁-C₄, formyle, (alkyl en C₁-C₄) -carbonyle, (alkoxy en C₁-C₄) carbonyle, (alkyl en C₁-C₄) aminocarbonyle, di-(alkyl en C₁-C₄) -aminocarbonyle, (alkyl en C₁-C₄) sulfonyle, halogéno (alkyl en C₁-C₄)sulfonyle, benzoyle ou phénylsulfonyle, les deux derniers groupes cités étant substitués par v restes pris parmi les groupes alkyle en C₁-C₄, halogénoalkyle en C₁-C₄, alkoxy en C₁-C₄, halogénoalkoxy en C₁-C₄, halogène, cyano et nitro ;
R⁸ représente un atome d'hydrogène, un groupe alkyle en C₁-C₄, alkoxy en C₁-C₄, alcényle en C₂-C₄, alcynyle en C₂-C₄, cycloalkyle en C₃-C₈,, aryle, arylalkyle en C₁-C₆, hétéroaryle, hétérocyclyle, halogénoalkyle en C₁-C₄ ;
R⁹ représente un atome d'hydrogène, un groupe alkyle en C₁-C₄, alcényle en C₂-C₄, alcynyle en C₂-C₄, cycloalkyle en C₃-C₉, aryle, arylalkyle en C₁-C₆, hétéroaryle, hétérocyclyle, halogéno-alkyle en C₁-C₄ ou, dans la mesure où R⁸ et R⁹ sont liés à un atome ou à deux atomes directement voisins, ils forment, ensemble avec les atomes qui les relient, un cycle à cinq ou à six chaînons saturé, partiellement ou complètement insaturé, qui présente p hétéroatomes pris dans le groupe comprenant un atome d'oxygène, d'azote et de soufre ;
Y représente une unité bivalente prise parmi les groupes 0, S, N-H, N-alkyle en C₁-C₄, CHR⁵ et C(R⁵)₂ ;
Z représente une unité bivalente prise parmi les groupes O, S, SO, SO₂, N-H, N-(alkyl en C₁-C₄), CHR⁵ et C(R⁵)₂ ;
m et n valent chacun 0, 1 ou 2;
o, p et q valent chacun 0 ou 1;
w et x valent chacun 0, 1, 2, 3 ou 4;
v vaut 0, 1, 2 ou 3

2. Benzoylcyclohexanediones selon la revendication 1, où
X¹ représente une unité bivalente prise parmi les groupes O, S et NH ;
R¹ représente un atome de chlore, de brome, de fluor, un groupe méthyle, éthyle, cyano, nitro, halogéno(alkyle en C₁-C₂) ;
R² représente un atome d'halogène, un groupe halogénoalkyle en C₁-C₄, (alkyl en C₁-C₄) - sulfényle, (alkyl en C₁-C₄) sulfinyle, (alkyl en C₁-C₄)sulfonyle ou nitro ;
R⁵ représente un groupe alkyle en C₁-C₄, cycloalkyle en C₃-C₈, alkoxy en C₁-C₄, (alkoxy en C₁-C₄)-alkyle en C₁-C₄, (alkyl en C₁-C₄) carbonyle, (alkoxy en C₁-C₄)carbonyle, (alkyl en C₁-C₄)thio, phényle, ou deux restes R⁵ liés à un atome de carbone commun forment une chaîne du groupe OCH₂CH₂O, OCH₂CH₂CH₂O, SCH₂CH₂S et SCH₂CH₂CH₂S, celle-ci étant substituée par w groupes méthyle, ou deux restes R⁵ liés à des atomes de carbone directement voisins forment une liaison ou forment, avec les atomes de carbone qui les portent, un cycle à 3 à 6 chaînons substitué par w restes pris dans le groupe comprenant halogène, alkyle en C₁-C₄, (alkyl en C₁-C₄) thio et alkoxy en C₁-C₄ ;
R⁸ représente un atome d'hydrogène, un groupe alkyle en C₁-C₄, alkoxy en C₁-C₄, alcényle en C₂-C₄, alcynyle en C₂-C₄, cycloalkyle en C₃-C₉, aryle, arylalkyle en C₁-C₆, halogénoalkyle en C₁-C₄ ;
R⁹ représente un atome d'hydrogène, un groupe alkyle en C₁-C₄ ou, dans la mesure où R⁸ et R⁹ sont liés à un atome ou à deux atomes directement voisins, ils forment ensemble avec les atomes qui les relient un cycle à cinq ou à six chaînons saturé, partiellement ou complètement insaturé, qui présente p hétéroatomes pris parmi les atomes d'oxygène, d'azote et de soufre.

3. Benzoylcyclohexanediones selon la revendication 1 ou 2, où
X² représente une chaîne alkylène en C₁-C₄, alcénylène en C₂-C₄ ou alcynylène en C₂-C₄ linéaire ou ramifiée, substituée par w atomes d'halogène ;
R³
a) représente un atome d'hydrogène, un groupe hydroxy, halogène, mercapto, amino, nitro, cyano, formyle,
b) représente un groupe phényle, oxazolyle, furanyle ou tétrahydropyrrolyle substitué par w restes du groupe constitué par un atome d'halogène, un groupe cyano, alkyle en C₁-C₄, halogénoalkyle en C₁-C₄, alkoxy en C₁-C₄, halogénoalkoxy en C₁-C₄, (alkyl en C₁-C₄) thio, halogéno (alkyl en C₁-C₄) thio et R¹⁰,
c) représente un groupe alkyle en C₁-C₆, alcényle en C₂-C₆, alcynyle en C₂-C₆, (alkoxy en C₁₋C₆) alkyle, (alcynyloxy en C₂-C₆)-alkyle en C₁-C₆, cycloalkyle en C₃-C₉, cycloalcényle en C₃-C₉, alkoxy en C₁-C₆ ou (alkyl en C₁-C₆) thio ; (R¹¹) (alkyl en C₁-C₄) amino, (R¹¹)₂amino, R¹¹⁻oxycarbonyle, R¹¹-carbonyle, R¹¹-carbonyloxy substitué par v restes pris parmi les groupes formyle, halogène, cyano, nitro, (alkyl en C₁₋C₄)amino, di(alkyl en C₁-C₄)amino, (alkoxy en C₁₋C₄) carbonyle, (alkyl en C₁-C₄)carbonyle, (alkyl en C₁-C₄) carbonyloxy, alkyle en C₁-C₄, alcényle en C₂-C₄, alcynyle en C₂-C₄, halogénoalkyle en C₁-C₄, (alkyl en C₁-C₄) thio, halogéno(alkyl en C₁-C₄) thio, alkoxy en C₁-C₄ et halogénoalkoxy en C₁- C₄ ;
d) un reste de formule Va, Vb, Vc, Vd, Vj ou Vp, ou
e) dans le cas où p vaut zéro, un groupe oxo, NR⁸, N-OR⁸ ou N-NR⁸R⁹
R⁷ représente un atome d'hydrogène, un groupe (alkyl en C₁-C₄) sulfonyle, benzoyle ou phényl-sulfonyle, les deux derniers groupes cités étant substitués par v restes pris dans parmi les groupes alkyle en C₁-C₂, halogénoalkyle en C₁-C₂, alkoxy en C₁-C₂, halogénoalkoxy en C₁-C₂, halogène, cyano et nitro, et
R¹¹ représente un atome d'hydrogène, un groupe alkyle en C₁-C₄, alcényle en C₂-C₄, alcynyle en C₂-C₄ ou cycloalkyle en C₃-C₈.

4. Benzoylcyclohexanediones selon l'une des revendications 1 à 3, où
X¹ représente une unité bivalente O ;
R⁴ représente un groupe OR⁷, (alkyl en C₁-C₄)thio, (alcényl en C₂-C₄)thio, (alkyl en C₁-C₄)-sulfonyle, cyano, cyanato, thiocyanato, ou un groupe phénylthio substitué par v restes pris dans le groupe comprenant halogène, alkyle en C₁-C₂, alkoxy en C₁-C₂, halogénoalkyle en C₁-C₂, halogénoalkoxy en C₁-C₂ et nitro, et
R⁵ représente un atome d'hydrogène, un groupe alkyle en C₁-C₄, cycloalkyle en C₃-C_{8;} alkoxy en C₁-C₄, (alkyl en C₁-C₄) thio, phényle, ou deux restes R⁵ liés à des atomes de carbone directement voisins forment, conjointement avec les atomes de carbone qui les portent, un cycle substitué à 3 à 6 chaînons ;
R¹² représente un atome d'hydrogène, un groupe alkyle en C₁-C₄, alcényle en C₂-C₄-, ou dans la mesure où R¹¹ et R¹² sont liés à un atome ou à deux atomes directement voisins, ils forment ensemble avec les atomes qui les relient un cycle saturé, partiellement ou complètement insaturé à cinq ou six chaînons, qui renferme p hétéroatomes pris parmi les atomes d'oxygène, d'azote et de soufre ;
Y représente une unité bivalente prise parmi les groupes CHR⁵ et C(R⁵)₂, et
Z représente une unité bivalente prise parmi les groupes O, S, SO₂, N-alkyle en C₁-C₄, CHR⁵ et C(R⁵)2.

5. Benzoylcyclohexanediones selon l'une des revendications 1 à 4, où
R² représente un atome d'halogène, un groupe halogénoalkyle en C₁-C₂ ou (alkyl en C₁-C₂)-sulfonyle ;
R⁵ représente un groupe alkyle en C₁-C₄, cycloalkyle en C₃-C₈, alkoxy en C₁-C₄, (alkyl en C₁-C₄) thio, phényle, ou deux restes R⁵ liés à des atomes de carbone directement voisins forment, avec les atomes de carbone qui les portent, un cycle substitué à 3 à 6 chaînons ;
R⁷ représente un atome d'hydrogène, un groupe (alkyl en C₁-C₄) sulfonyle, benzoyle ou phénylsulfonyle, et
R⁸ représente un atome d'hydrogène, un groupe méthyle ou éthyle, et
R² se trouve en position 4 du cycle phényle.

6. Bénzoylcyclohexariediones selon l'une des revendications 1 à 5, où
X² représente une chaîne alkylène en C₁-C₄, alcénylène en C₂-C₄ ou alcynylène en C₂-C₄ linéaire ou ramifiée ;
R¹ représente un atome de chlore, de brome, un groupe méthyle, trifluorométhyle, cyano ou nitro ;
R² représente un atome de chlore, de brome, un groupe méthylsulfonyle, éthylsulfonyle, trifluorométhyle ou nitro ;
R⁴ représente un groupe OR⁷, (alkyl en C₁-C₄)thio, (alcényl en C₂-C₄)thio ou phénylthio ;
R⁵ représente un atome d'hydrogène, un groupe alkyle en C₁-C₄, ou deux restes R⁵ liés à des atomes de carbone directement voisins forment, avec les atomes de carbone qui les portent, un cycle substitué à 3 à 6 chaînons ;
A représente une unité bivalente prise parmi les groupes O, S(O)ₙ, NH et alkyle en C₁-C₆ ;
M représente un groupe alkylène en C₁-C₆ ;
Y et Z, indépendamment l'un de l'autre, représentent une unité bivalente prises dans le groupe de CHR⁵ et C(R⁵)_{2.}

7. Agents herbicides **caractérisés par** une teneur à efficacité herbicide en au moins un composé de formule générale (I) selon l'une des revendications 1 à 6.

8. Agents herbicides selon la revendication 7 en mélange avec des formulants.

9. Procédé pour la lutte contre les plantes indésirables, **caractérisé en ce qu'**on applique une quantité efficace d'au moins un composé de formule générale (I) selon l'une des revendications 1 à 6 ou d'un agent herbicide selon la revendication 7 ou 8, aux plantes ou au site de la végétation indésirable.

10. Utilisation de composés de formule générale (I) selon l'une des revendications 1 à 6 ou d'agents herbicides selon la revendications 7 ou 8 pour la lutte contre les plantes indésirables.

11. Utilisation selon la revendication 10, **caractérisée en ce que** les composés de formule générale (I) sont utilisés pour la lutte contre les plantes indésirables dans les cultures de plantes utiles.

12. Utilisation selon la revendication 11, **caractérisée en ce que** les plantes utiles sont des plantes utiles transgéniques.
